# EUROPEAN PATENT APPLICATION

(11) **EP 4 609 799 A2**
(43) Date of publication of application: **03.09.2025**
(21) Application number: 25179277.6
(22) Date of filing: 30.12.2020
(51) Int. Cl.: A61B 8/00

(54) **ULTRASOUND FINGER PROBE AND METHODS THEREOF**

(30) Priority: 03.01.2020 US 202062957003 P
(62) Divisional of application: 20845351.4
(71) Applicant: Bard Access Systems, Inc., Salt Lake City, UT 84116 (US)
(72) Inventor: ROBBINS, Tab, Layton, UT, 84041 (US); PRINCE, Matthew J., Herriman, UT, 84096 (US); PETERSON, Bart, Farmington, UT, 84025 (US); WESTWOOD, Paul T., Kaysville, UT, 84037 (US); OROME, Amir, Sandy, UT, 84093 (US)
(74) Representative: Hoffmann Eitle

(57) **Abstract**

Ultrasound probes for use in an ultrasound imaging system are disclosed. The ultrasound probes are sized and configured so as to be supported and readily used with as little as one finger on a single hand of a user of the ultrasound imaging system. This configuration enables remaining fingers on the hand of the user to be employed for other purposes. **In** one embodiment, for example, an ultrasound probe is disclosed including a body, a lens included on head portion of the body, a stabilizing portion extending from the body and configured to stabilize the body on a skin surface of a patient without user assistance, and a finger-grip portion configured to enable a user of the ultrasound probe to grasp and maneuver the ultrasound probe during use thereof with no more than two or three fingers on a single hand of the user.

## Description

This application claims the benefit of U.S. Provisional Application No. 62/957,003, filed January 3, 2020, which is incorporated by reference in its entirety into this application.

### BACKGROUND

Ultrasound probes can be large, bulky, and cumbersome to manipulate, which, in turn, can make it difficult to image desired tissues. Comparatively small ultrasound probes are available, but such ultrasound probes can also make it difficult to image desired tissues due to their diminutive size and lack of features for manipulating the ultrasound probes or applying sufficient pressure therewith for proper acoustic coupling.

Discloses herein are ultrasound finger ultrasound probes and methods there of that address at least the foregoing.

### SUMMARY

Briefly summarized, disclosed herein are ultrasound probes for use in an ultrasound imaging system. The ultrasound probes are sized and configured so as to be supported and readily used with as little as one finger on a single hand of a user of the ultrasound imaging system such as a clinician. This configuration enables remaining fingers on the hand of the user to be employed for other purposes including applying traction to the skin surface of the patient proximate the imaging site and providing touch comfort to the patient. With ultrasound probes supported by as little as a single finger, the ultrasound probes can be easily positioned by the user in a proper orientation against the skin surface during imaging procedures.

Disclosed herein is an ultrasound probe including, in some embodiments, a body, a lens, a stabilizing portion of the body, and a finger-grip portion of the body. The lens is disposed in a cutout of the body designating a head portion of the ultrasound probe. The lens is configured to pass ultrasound signals therethrough. The stabilizing portion is composed of a longitudinal extension of the body extending away from the head portion of the ultrasound probe. The stabilizing portion of the ultrasound probe is configured to stabilize the ultrasound probe on a skin surface of a patient without assistance from a user of the ultrasound probe. The finger-grip portion is composed of a generally transverse extension of the body extending away from the head portion of the ultrasound probe. The finger-grip portion of the ultrasound probe is configured to enable the user to grasp and maneuver the ultrasound probe during use thereof with no more than two or three fingers of a single hand.

In some embodiments, the ultrasound probe further includes a cable conduit composed of a curved extension of the body extending away from both the stabilizing and head portions of the ultrasound probe. The cable conduit is configured to house a distal-end portion of a power-and-data cable for connecting the ultrasound probe to a console. The cable conduit is also configured to keep the power-and-data cable away from the head portion of the ultrasound probe.

In some embodiments, the ultrasound probe further includes a cable boot having a distal-end portion disposed in an opening of the cable conduit. The cable boot is configured to house the distal-end portion of the power-and-data cable proximal of the cable conduit. The cable boot is also configured to keep the power-and-data cable farther away from the head portion of the ultrasound probe.

In some embodiments, the cable boot is configured to attenuate a bending radius of the power-and-data cable about the opening of the cable conduit. Attenuating the bending radius of the power-and-data cable reduces a risk of damaging the power-and-data cable at the opening of the cable conduit where the power-and-data cable is most likely to overbend.

In some embodiments, the stabilizing portion of the ultrasound probe includes a latitudinal channel extending from side to side of two longitudinal sides of the ultrasound probe. The latitudinal channel reduces a surface area of a generally flat surface of the stabilizing portion intended to face the skin surface of the patient.

In some embodiments, the surface area of the stabilizing portion of the ultrasound probe reduced by the lateral channel facilitates movement of the ultrasound probe across the skin surface of the patient by reducing contact area between the surface of the stabilizing portion and the skin surface of the patient.

In some embodiments, the surface area of the stabilizing portion of the ultrasound probe reduced by the lateral channel reduces suction between the surface of the stabilizing portion and the skin surface of the patient when gel is therebetween and the ultrasound probe is removed from the patient.

In some embodiments, the finger-grip portion of the ultrasound probe includes a concave surface opposite a generally flat surface.

In some embodiments, the concave surface of the finger-grip portion is an extension of a concave surface of the stabilizing portion of the ultrasound probe intended to face away from the skin surface of the patient.

In some embodiments, the ultrasound probe is bilaterally symmetrical about a plane of symmetry of the ultrasound probe.

In some embodiments, at least a portion of the body is molded thermoplastic coupled together along the plane of symmetry of the ultrasound probe.

Also disclosed herein is an ultrasound system including, in some embodiments, a console and an ultrasound probe. The console is configured for processing and depicting ultrasound images on a display of the console. The ultrasound probe includes body, a lens, a stabilizing portion of the body, a cable conduit, and a finger-grip portion of the body. The lens is disposed in a cutout of the body designating a head portion of the ultrasound probe. The lens is configured to pass ultrasound signals therethrough. The stabilizing portion is composed of a longitudinal extension of the body extending away from the head portion of the ultrasound probe. The stabilizing portion of the ultrasound probe is configured to stabilize the ultrasound probe on a skin surface of a patient without assistance from a user of the ultrasound probe. The cable conduit is composed of a curved extension of the body extending away from both the stabilizing and head portions of the ultrasound probe. The cable conduit is configured to house a distal-end portion of a power-and-data cable for connecting the ultrasound probe to the console. The finger-grip portion is composed of a generally transverse extension of the body extending away from the head portion of the ultrasound probe. The finger-grip portion of the ultrasound probe is configured to enable the user to grasp and maneuver the ultrasound probe during use thereof with no more than two or three fingers of a single hand.

Also disclosed herein is a method of an ultrasound probe including, in some embodiments, an obtaining step of obtaining the ultrasound probe. The ultrasound probe includes a body, a stabilizing portion of the body, and a finger-grip portion of the body. The body has a lens disposed in a cutout of the body designating a head portion of the ultrasound probe. The stabilizing portion is composed of a longitudinal extension of the body extending away from the head portion of the ultrasound probe. The finger-grip portion composed of a generally transverse extension of the body extending away from the head portion of the ultrasound probe. The method also includes a placing step of placing the head and stabilizing portions of the ultrasound probe on a skin surface of a patient. The method also includes an enabling step of enabling probing ultrasound signals to be emitted into the patient from an array of piezoelectric transducers within the ultrasound probe for ultrasound imaging. The method also includes a moving step of moving the ultrasound probe for the ultrasound imaging by the finger-grip portion of the ultrasound probe using no more than two or three fingers of a single hand.

In some embodiments, the method also includes a grasping step of grasping the ultrasound probe before the moving step. The grasping step includes placing at least one of an index finger or a middle finger on a concave surface of the finger-grip portion of the ultrasound probe and a thumb on an opposing, generally flat surface of the finger-grip portion of the ultrasound probe.

In some embodiments, the grasping step orients a palm of the single hand substantially parallel to the skin surface of the patient.

In some embodiments, the method also includes an applying step of applying an ultrasound gel to the skin surface of the patient before the placing step.

In some embodiments, the method also includes a removing step of removing the ultrasound probe from the skin surface of a patient. The stabilizing portion of the ultrasound probe includes a latitudinal channel extending from side to side of two longitudinal sides of the ultrasound probe. The latitudinal channel reduces a surface area of a generally flat surface of the stabilizing portion, thereby reducing gel-induced suction between the surface of the stabilizing portion and the skin surface of the patient during the removing step.

In some embodiments, the method also includes a connecting step of connecting a power-and-data cable of the ultrasound probe to a console of an ultrasound imaging system. The cable extends from a cable conduit of the ultrasound probe composed of a curved extension of the body extending away from both the stabilizing and head portions of the ultrasound probe. The cable conduit is configured to keep the power-and-data cable away from the head portion of the ultrasound probe.

In some embodiments, the moving step does not overbend the power-and-data cable due to attenuation of a bending radius of the power-and-data cable by a cable boot. The cable boot has a distal-end portion disposed in an opening of the cable conduit from which the power-and-data cable extends.

In some embodiments, the method also includes another enabling step of enabling reflected ultrasound signals to be reflected from the patient into the array of piezoelectric transducers within the ultrasound probe for conversion into electrical signals for corresponding ultrasound images.

These and other features of the concepts provided herein will become more apparent to those of skill in the art in view of the accompanying drawings and following description, which describe particular embodiments of such concepts in greater detail.

### DRAWINGS

FIG. 1 provides a perspective view of an ultrasound imaging system including an ultrasound probe in accordance with some embodiments.
FIG. 2 provides a block diagram of the ultrasound imaging system of FIG. 1.
FIG. 3A provides a perspective view of a first ultrasound probe in accordance with some embodiments.
FIG. 3B provides another perspective view of the first ultrasound probe in accordance with some embodiments.
FIG. 3C provides a front view of the first ultrasound probe in accordance with some embodiments.
FIG. 3D provides a side view of the first ultrasound probe in accordance with some embodiments.
FIG. 4A provides a perspective view of a second ultrasound probe in accordance with some embodiments.
FIG. 4B provides another perspective view of the second ultrasound probe in accordance with some embodiments.
FIG. 4C provides a front view of the second ultrasound probe in accordance with some embodiments.
FIG. 4D provides a side view of the second ultrasound probe in accordance with some embodiments.
FIG. 5A provides a perspective view of a third ultrasound probe in accordance with some embodiments.
FIG. 5B provides another perspective view of the third ultrasound probe in accordance with some embodiments.
FIG. 5C provides a front view of the third ultrasound probe in accordance with some embodiments.
FIG. 5D provides a side view of the third ultrasound probe in accordance with some embodiments.
FIG. 6A provides a perspective view of a fourth ultrasound probe in accordance with some embodiments.
FIG. 6B provides another perspective view of the fourth ultrasound probe in accordance with some embodiments.
FIG. 6C provides a front view of the fourth ultrasound probe in accordance with some embodiments.
FIG. 6D provides a side view of the fourth ultrasound probe in accordance with some embodiments.
FIG. 6E provides a rear view of the fourth ultrasound probe in accordance with some embodiments.
FIG. 7A provides a perspective view of a fifth ultrasound probe in accordance with some embodiments.
FIG. 7B provides another perspective view of the fifth ultrasound probe in accordance with some embodiments.
FIG. 7C provides a front view of the fifth ultrasound probe in accordance with some embodiments.
FIG. 7D provides a side view of the fifth ultrasound probe in accordance with some embodiments.
FIG. 8A provides a perspective view of a sixth ultrasound probe in accordance with some embodiments.
FIG. 8B provides another perspective view of the sixth ultrasound probe in accordance with some embodiments.
FIG. 8C provides a front view of the sixth ultrasound probe in accordance with some embodiments.
FIG. 8D provides a side view of the sixth ultrasound probe in accordance with some embodiments.
FIG. 9A provides a perspective view of a seventh ultrasound probe in accordance with some embodiments.
FIG. 9B provides another perspective view of the seventh ultrasound probe in accordance with some embodiments.
FIG. 9C provides a front view of the seventh ultrasound probe in accordance with some embodiments.
FIG. 9D provides a side view of the seventh ultrasound probe in accordance with some embodiments.
FIG. 10A provides a perspective view of an eighth ultrasound probe in accordance with some embodiments.
FIG. 10B provides another perspective view of the eighth ultrasound probe in accordance with some embodiments.
FIG. 10C provides a front view of the eighth ultrasound probe in accordance with some embodiments.
FIG. 10D provides a side view of the eighth ultrasound probe in accordance with some embodiments.
FIG. 11A provides a perspective view of a ninth ultrasound probe in accordance with some embodiments.
FIG. 11B provides another perspective view of the ninth ultrasound probe in accordance with some embodiments.
FIG. 11C provides a front view of the ninth ultrasound probe in accordance with some embodiments.
FIG. 11D provides a side view of the ninth ultrasound probe in accordance with some embodiments.
FIG. 12A provides a perspective view of a tenth ultrasound probe in accordance with some embodiments.
FIG. 12B provides another perspective view of the tenth ultrasound probe in accordance with some embodiments.
FIG. 12C provides a front view of the tenth ultrasound probe in accordance with some embodiments.
FIG. 12D provides a side view of the tenth ultrasound probe in accordance with some embodiments.
FIG. 13A provides a perspective view of an eleventh ultrasound probe in accordance with some embodiments.
FIG. 13B provides another perspective view of the eleventh ultrasound probe in accordance with some embodiments.
FIG. 13C provides a side view of the eleventh ultrasound probe in accordance with some embodiments.
FIG. 13D provides a rear view of the eleventh ultrasound probe in accordance with some embodiments.
FIG. 14 provides a perspective view of a twelfth ultrasound probe in accordance with some embodiments.
FIG. 15A provides a perspective view of a thirteenth ultrasound probe in accordance with some embodiments.
FIG. 15B provides a side view of the thirteenth ultrasound probe in accordance with some embodiments.
FIG. 16A provides a perspective view of a fourteenth ultrasound probe in accordance with some embodiments.
FIG. 16B provides another perspective view of the fourteenth ultrasound probe in accordance with some embodiments.
FIG. 16C provides a side view of the fourteenth ultrasound probe in accordance with some embodiments.
FIG. 16D provides a rear view of the fourteenth ultrasound probe in accordance with some embodiments.
FIG. 17 provides a perspective view showing a hand of a user engaged with the second ultrasound probe in accordance with some embodiments.
FIG. 18A provides a first side view of a fifteenth ultrasound probe in accordance with some embodiments.
FIG. 18B provides a second side view of the fifteenth ultrasound probe in accordance with some embodiments.
FIG. 18C provides a top view of the fifteenth ultrasound probe in accordance with some embodiments.
FIG. 18D provides a bottom view of the fifteenth ultrasound probe in accordance with some embodiments.
FIG. 18E provides a rear view of the fifteenth ultrasound probe in accordance with some embodiments.
FIG. 18F provides a front view of the fifteenth ultrasound probe in accordance with some embodiments.
FIG. 18G provides a perspective view of the fifteenth ultrasound probe in accordance with some embodiments.
FIG. 19 provides a perspective view of a sixteenth ultrasound probe in accordance with some embodiments.
FIG. 20A provides a perspective view of a seventeenth ultrasound probe in accordance with some embodiments.
FIG. 20B provides another perspective view of the seventeenth ultrasound probe in accordance with some embodiments.
FIG. 21A provides a perspective view of an eighteenth ultrasound probe in accordance with some embodiments.
FIG. 21B provides another perspective view of the eighteenth ultrasound probe in accordance with some embodiments.
FIG. 22A provides a perspective view of a nineteenth ultrasound probe in accordance with some embodiments.
FIG. 22B provides another perspective view of the nineteenth ultrasound probe in accordance with some embodiments.
FIG. 23A provides a perspective view of a twentieth ultrasound probe in accordance with some embodiments.
FIG. 23B provides another perspective view of the twentieth ultrasound probe in accordance with some embodiments.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal-end portion" of, for example, a catheter disclosed herein includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal-end portion, or the proximal length of the catheter can include the proximal end of the catheter; however, the proximal portion, the proximal-end portion, or the proximal length of the catheter need not include the proximal end of the catheter. That is, unless context suggests otherwise, the proximal portion, the proximal-end portion, or the proximal length of the catheter is not a terminal portion or terminal length of the catheter.

With respect to "distal," a "distal portion" or a "distal-end portion" of, for example, a catheter disclosed herein includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal-end portion, or the distal length of the catheter can include the distal end of the catheter; however, the distal portion, the distal-end portion, or the distal length of the catheter need not include the distal end of the catheter. That is, unless context suggests otherwise, the distal portion, the distal-end portion, or the distal length of the catheter is not a terminal portion or terminal length of the catheter.

Unless defined otherwise, all technical and scientific terms used herein have the same meaning as commonly understood by those of ordinary skill in the art.

Embodiments disclosed herein are generally directed to an ultrasound probe for use with an ultrasound imaging system. In particular, the ultrasound probe is sized and configured so as to be supported and readily used with one or more fingers on a single hand of a user of the ultrasound imaging system, such as a clinician. This configuration enables remaining fingers on the hand of the user to be employed for other purposes, including applying traction to the skin surface of the patient proximate the imaging site, providing touch comfort to the patient, etc. With it supported by as little as a single finger, the ultrasound probe can easily be positioned by the user in a proper orientation against the skin surface during imaging procedures.

Further, the ultrasound probe is configured in one embodiment to enable the user's hand to be positioned substantially horizontally with respect to (i.e., parallel to) the skin surface of the patient, thus enabling relatively accurate ultrasound-probe positioning (e.g., lifting, placing, pressing, etc.) and scanning operations to be performed.

In one embodiment, the ultrasound probe also includes a stabilizing portion to assist in maintaining stability of the ultrasound probe while on the skin surface.

In one embodiment, the ultrasound probe is configured to be supported and manipulated by a single finger, with the finger positioned directly over a lens portion of the ultrasound probe. Such a position enables the user to easily and accurately move the ultrasound probe on the skin surface as to position the ultrasound probe with subtle movements as desired.

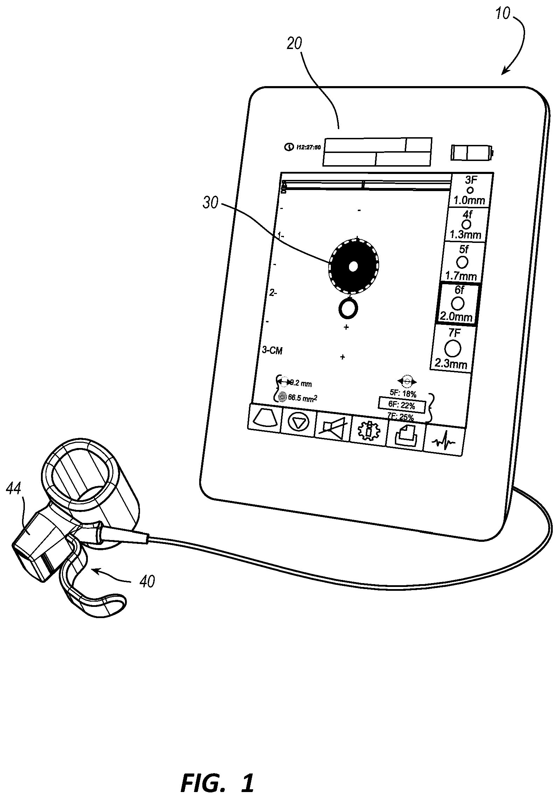

FIG. 1 shows various components of an ultrasound imaging system 10 according to one embodiment. As shown, the ultrasound imaging system 10 includes a console 20 housing various electronic and other components necessary for processing and depicting ultrasonic images. The console 20 includes a touchscreen display 30 for depicting ultrasonic images and for enabling touch-based input by a clinician to control the device and its functionality. An ultrasound probe 40, containing one or more transducer elements in a head 44 thereof for emitting and receiving ultrasonic signals, is operably attached to the console 20 via a cable or other suitable interface, including wireless connectivity.

In one embodiment, an optional cap including a hydrogel insert can be removably attached to the head 44 of the ultrasound probe 40 so as to cover a lens portion thereof. The hydrogel insert provides an ultrasonically transparent interface between the ultrasound-probe head 44 and the skin surface. A needle guide can also be included with the cap to assist with guiding needles through the patient's skin and into the vessel being imaged by the ultrasound imaging system 10. In another embodiment, the needle guide is included on the ultrasound probe itself. Further details regarding the ultrasound-probe cap, hydrogel insert, and needle guide can be found in U.S. Publication No. 2011/0313293 and U.S. Patent No. 9,788,812, each of which is incorporated herein by reference in its entirety. In yet another embodiment, a sheath or cover can be removably placed over the ultrasound probe 40 to provide a sterile barrier. Note that other ultrasound imaging devices and ultrasound imaging systems that differ from that shown here can also benefit from the embodiments described herein.

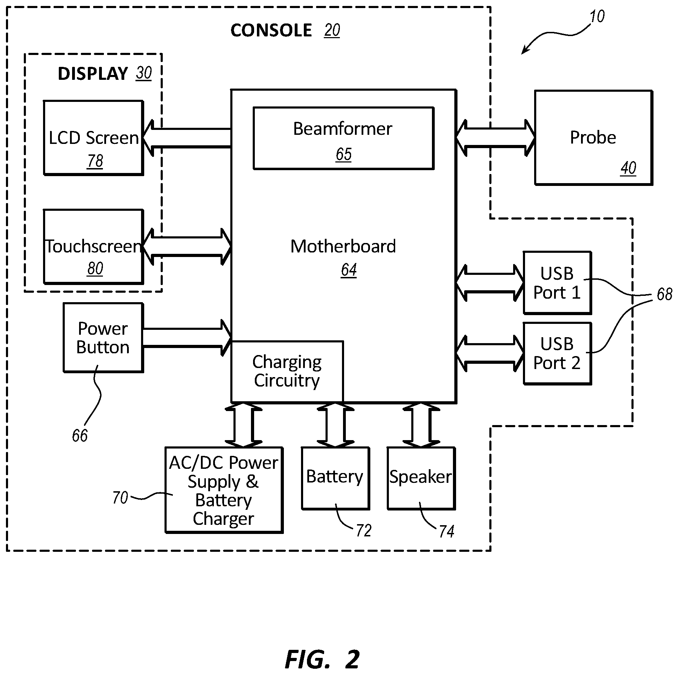

FIG. 2 shows a block diagram of the ultrasound imaging system 10 of FIG. 1, according to one embodiment. In detail, the console 20, display 30, and ultrasound probe 40 are represented, as in FIG. 1. The console 20 includes therein a motherboard 64 for governing system functionality and includes a processor or other general or special purpose computer, memory, storage locations, and other components for system operation. A beamformer 65, including suitable circuitry, is also operably included with the motherboard 64 to enable ultrasonic signals to be produced, received, and processed. A power button 66 is included, as are USB ports 68 for interfacing with other devices. An external power supply 70, as well as a battery 72 and speaker 74, are provided for operation. The display 30 in the present embodiment includes an LCD screen 78 or other suitable screen, and a touchscreen 80 to enable touch-based functionality via the display 30. Note that the ultrasound imaging system 10 can include different, fewer, or more components than those listed here, including those components that enable the ultrasound imaging system to operate in a networked manner with other local or remote computing or network systems, including for instance, Wi-Fi, Ethernet, Bluetooth, and ZigBee functionality. Also, in addition to a touchscreen, other input modes can also be employed, including a keyboard or mouse input, for instance.

In operation of the ultrasound imaging system 10, a lens portion of the head 44 of the ultrasound probe 40 is placed against the skin of the patient so as to ultrasonically image a cross-sectional slice of a vessel, such as a vein, or other internal body tissue of the patient below the surface of the skin. Indeed, a target location of the vessel imaged by the ultrasound probe 40 is disposed a substantially vertical depth below the end of the ultrasound probe. The vessel is imaged by the ultrasound imaging system 10 in preparation for accessing the vessel with a needle in preparation for inserting a catheter into the vessel, in one embodiment. Though shown here as a vessel, the target location can be any one of various subcutaneous locations within the body.

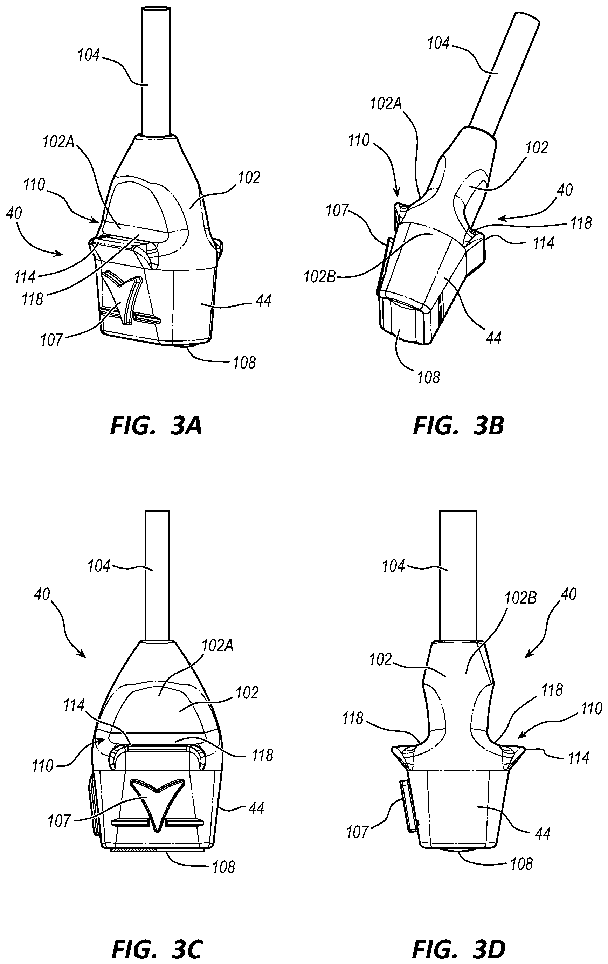

FIGS. 3A-3D depict various features of the ultrasound probe 40 including the head 44 thereof, in accordance with one embodiment. As shown, the ultrasound probe 40 includes a body 102 defining lateral surfaces 102A and end surfaces 102B, with the head 44 of the ultrasound probe defining a distal end of the ultrasound-probe body. A lens 108 is disposed at the distal end of the ultrasound probe body 102 and is configured to enable passage therethrough of ultrasonic signals. An orientation arrow 107 is included in the illustrated embodiment to assist a user of the ultrasound imaging system in determining proper needle placement through the skin surface of a patient. A cable 104 is shown extending from a proximal end of the ultrasound probe body 102 to operably connect the ultrasound probe to the console 20 (FIG. 1), though the cable can extend from the ultrasound probe in accordance with various different configurations.

In the present embodiment, the ultrasound probe 40 includes a finger-grip portion 110 configured to enable the user of the ultrasound probe to grasp, support, and handle the ultrasound probe with one or more fingers of a single hand of the user while leaving one or more fingers of the user's hand free to be used in other ways. In the present embodiment, the finger-grip portion 110 includes two angled, protruding elements 114 extending from the opposing lateral surfaces 102A of the ultrasound-probe body 102. Each of the protruding elements 114 defines, together with a proximate portion of the lateral surface 102A of the ultrasound-probe body 102, a concavely shaped grip surface 118 into which a user's finger is received in order to support and move the ultrasound probe 40 during system operation.

In the present embodiment, the ultrasound probe 40 is placed between a forefinger (index finger) and a middle finger of a single hand of the user such that an inside portion of the forefinger engages one of the grip surfaces 118 and an inside portion of the middle finger engages the other grip surface on the opposing side of the ultrasound probe. In this way, the user is able to lift, maneuver, slide, and otherwise move the ultrasound probe 40 with only two fingers during operation of the ultrasound imaging system 10. Note that other fingers of the user's hand can alternatively be used to hold the ultrasound probe 40. This further enables the remaining three fingers of the user's hand to be employed in other ways during ultrasonic imaging by the ultrasound probe 40. These other ways include, for instance, applying traction to the skin surface, touching the patient in order to impart comfort, establishing or marking a physical reference point etc.

Note that the engagement of the ultrasound probe 40 by the hand of the user is such that the user's hand is positioned substantially parallel with respect to the skin surface of the patient, i.e., horizontally, in a typical imaging procedure. This in turn enables the user to move the ultrasound probe accurately and easily, with relatively small movements across the skin surface, which results in improved imaging results. Note also that the ultrasound-probe body 102 in this and other embodiments includes a suitable material, such as a thermoplastic. In one embodiment, the material includes R-5100 polyphenylsulfone.

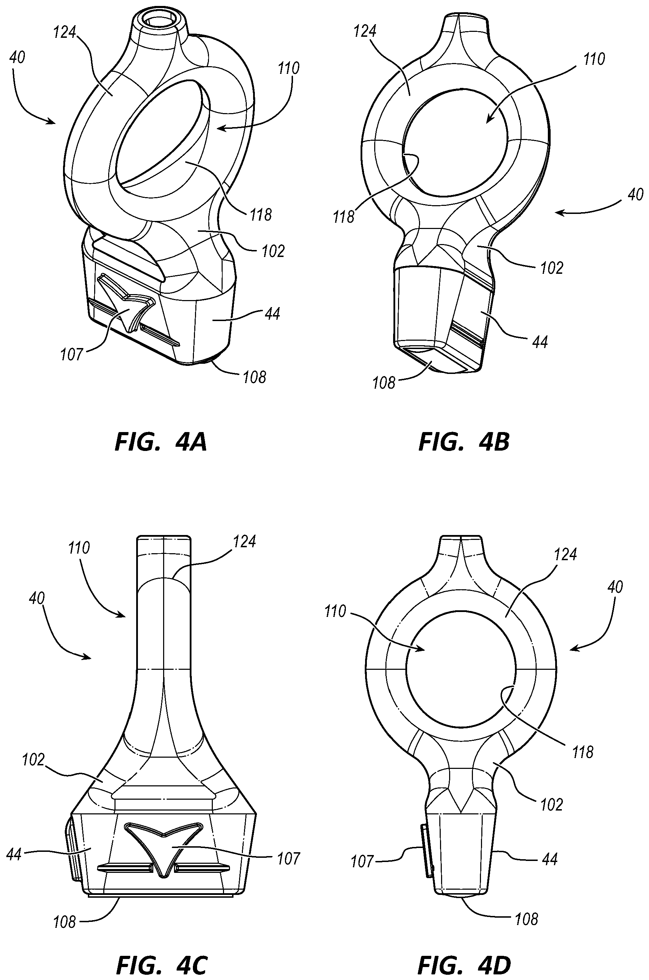

Reference is now made to FIGS. 4A-4D, which depict various details of the ultrasound probe 40 and its finger-grip portion 110 for enabling grasping, supporting, and handling of the ultrasound probe with one or more fingers of a single hand of the user while leaving one or more fingers of the user's hand free to be used in other ways as described herein, according to another embodiment. As shown, the finger-grip portion 110 includes a ring 124 defined by the body 102 of the ultrasound probe 40. The ring 124 is sized in one embodiment to receive a finger of the user, such as the forefinger or middle finger. To that end, an inner annular surface of the ring 124 serves as a grip surface 118 against which the user's finger rests to enable the finger to handle and control movement of the ultrasound probe 40. It is thus noted that the finger-grip portion 110, i.e., the ring 124 in the present embodiment, further serves as one example of a retention portion to retain engagement of the ultrasound probe 40 with the user's finger(s). Note that, in this and other embodiments, the retention portion enables the ultrasound probe 40 to remain attached with the finger of the use even when the finger and/or hand of the user is lifted from the skin surface, which enables the user to perform other tasks without putting down the ultrasound probe.

In one embodiment, the size of the ring 124 can be configured such that the user's finger can be inserted varying distances through the ring in order to encounter a fit suitable for moving and handling the ultrasound probe 40. As such, the ring 124 can be sized in one or more of varying diameters. In another embodiment, an insert can be removably fitted in to the ring 124 to adjust the size of the ring opening to accommodate differently sized fingers. Note that in one embodiment, all or part of the finger-grip portion can be flexible/resilient so as to deform to a user's finger. Note also, that the ultrasound probe 40 in the embodiment illustrated in FIGS. 4A-4D is about 2.5 inches high, the ultrasound-probe head 44 about 1 inch wide, and the ring 124 about 1.5 inches wide, though other dimensions are possible for this and the other embodiments described herein.

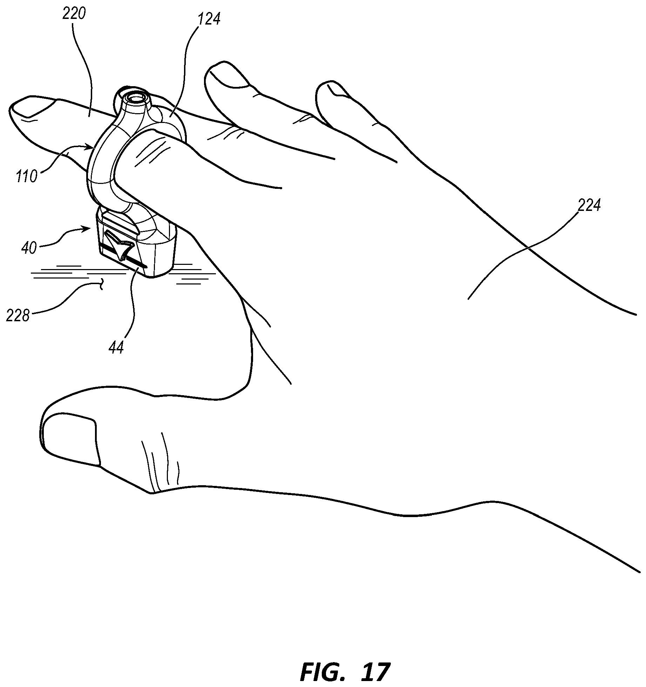

FIG. 17 shows the ultrasound probe 40 of FIGS. 4A-4D engaged with a forefinger 220 of a hand 224 of a user in one use embodiment. As shown, the finger-grip portion 110, i.e., the ring 124, enables the user's finger to be positioned substantially co-linearly above and in a spaced-apart relationship with respect to the lens 108 of the ultrasound-probe head 44 when the ultrasound probe is in an operable position on a horizontal skin surface of the patient 228. This enables the user to impart relatively fine amounts of pressure, or traction, to the skin surface via pressing down the ultrasound probe 40 thereon, and provides enhanced control over the ultrasound probe. Also shown in FIG. 17 is the manner in which the hand of the user is positioned substantially parallel with respect to the skin surface of the patient, i.e., horizontally, in a typical imaging procedure. As mentioned, this enables the user to move the ultrasound probe accurately and easily, with relatively small movements across the skin surface, which results in improved imaging results.

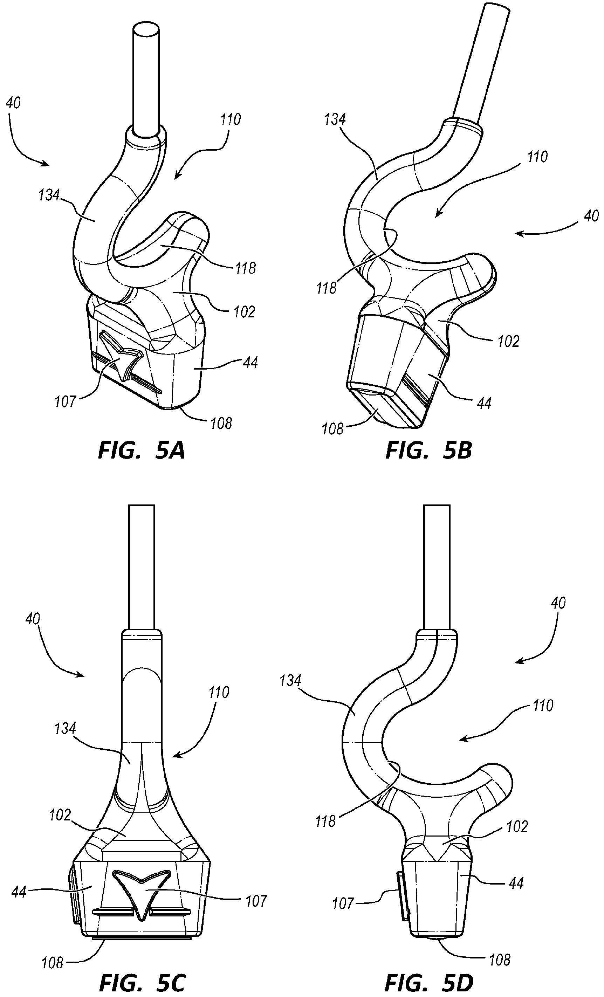

Reference is now made to FIGS. 5A-5D, which depict various details of the ultrasound probe 40 and its finger-grip portion 110 for enabling grasping, supporting, and handling of the ultrasound probe with one or more fingers of a single hand of the user while leaving one or more fingers of the user's hand free to be used in other ways, according to another embodiment. As shown, the finger-grip portion 110 includes a hook 134 defined by the body 102 of the ultrasound probe 40. The hook 134 is approximately C-shaped and is sized in one embodiment to receive a finger of the user, such as the forefinger or middle finger, while enabling an opening for ease of finger insertion/removal. An inner surface of the hook 134 serves as the grip surface 118 against which the user's finger rests to enable the finger to handle and control movement of the ultrasound probe 40. It is thus noted that the finger-grip portion 110, i.e., the hook 134 in the present embodiment, further serves as one example of a retention portion to retain engagement of the ultrasound probe 40 with the user's finger(s).

In one embodiment, the size of the hook 134 can be configured such that different portions of the user's finger can be inserted into the hook in order to encounter a fit suitable for moving and handling the ultrasound probe 40. As such, the hook 134 can be sized in one or more of varying sizes. In another embodiment, an insert can be removably fitted in to the hook 134 to adjust the size thereof to accommodate differently sized fingers.

The finger-grip portion 110, i.e., the hook 134 in the embodiment of FIGS. 5A-5D, enables the user's finger to be positioned substantially co-linearly above and in a spaced-apart relationship with respect to the lens 108 of the ultrasound-probe head 44. This enables the user to impart relatively fine amounts of pressure, or traction, to the skin surface via pressing down the ultrasound probe 40 thereon, and provides enhanced control over the ultrasound probe.

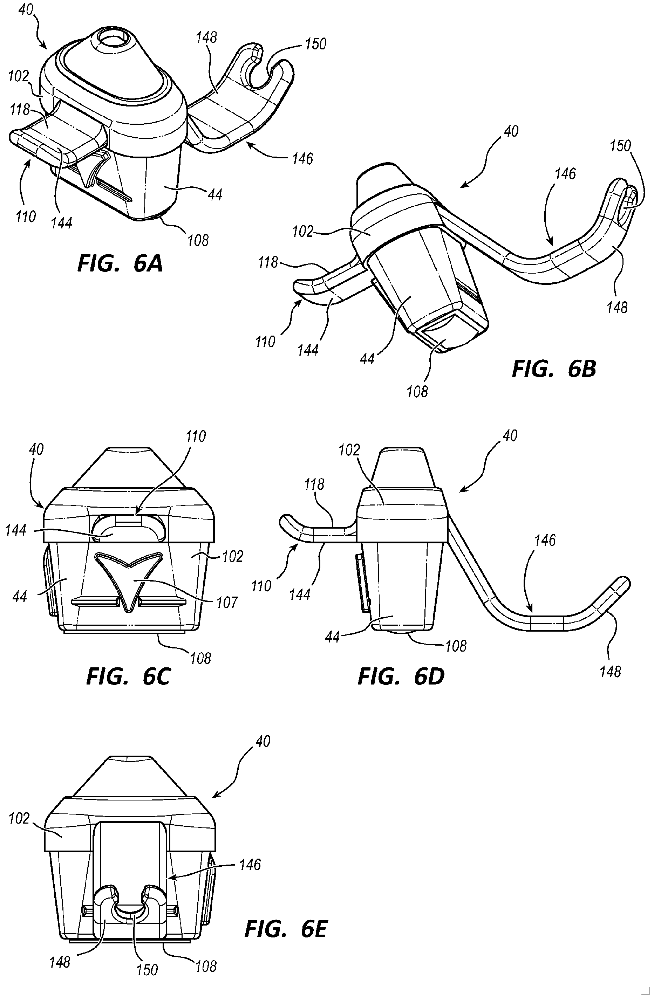

Reference is now made to FIGS. 6A-6E, which depict various details of the ultrasound probe 40 and its finger-grip portion 110 for enabling grasping, supporting, and handling of the ultrasound probe with one or more fingers of a single hand of the user while leaving one or more fingers of the user's hand free to be used in other ways, according to another embodiment. As shown, the finger-grip portion 110 includes a tab 144 extending from one of the lateral surfaces 102A of the ultrasound-probe body 102. A terminal portion of the tab 144 includes an upwardly angled lip so as to provide the grip surface 118 on which a finger of the user, such as the forefinger, can rest. Another finger, such as the middle finger on the same hand of the user, can engage the opposing lateral surface 102A of the ultrasound probe body 102 to enable the two fingers to handle and control movement of the ultrasound probe 40. Note that other fingers can be employed to grasp the ultrasound probe 40, as with the other embodiments herein.

The ultrasound probe 40 of the embodiment of FIGS. 6A-6E further includes a stabilizing portion 146 configured to maintain the ultrasound probe in an upright, usable position on the skin surface of the patient even when the user's hand is not contacting the ultrasound probe. In the present embodiment, the stabilizing portion 146 includes an angled leg 148 extending from the lateral surface 102A opposite the tab 144, best seen in FIG. 6D. The leg 148 extends downward (from the perspective shown in FIG. 6D) so as to provide a lower surface substantially flush with the lens 108 of the ultrasound-probe head 44. In this way, the leg 148 stabilizes the ultrasound probe 40 by providing a second contacting surface for the skin, in addition to the lens 108. Note that the stabilizing portion 146 can also enable the ultrasound probe 40 to be manipulated by a single finger, in one embodiment. A terminal free end of the leg 148 defines a notch 150, in the present embodiment, the notch configured for removably holding a portion of the cable 104 to keep it away from the hand of the user during use of the ultrasound probe 40.

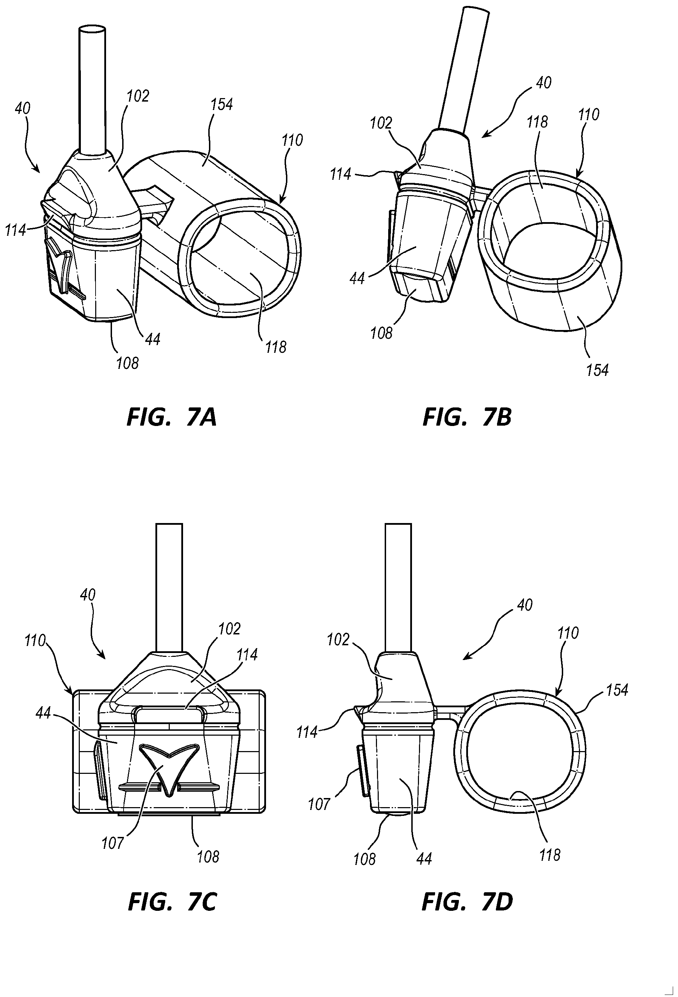

Reference is now made to FIGS. 7A-7D, which depict various details of the ultrasound probe 40 and its finger-grip portion 110 for enabling grasping, supporting, and handling of the ultrasound probe with one or more fingers of a single hand of the user while leaving one or more fingers of the user's hand free to be used in other ways, according to another embodiment. As shown, the finger-grip portion 110 includes a cylindrical ring 154 extending from the main body 102 of the ultrasound probe 40. The cylindrical ring 154 is sized in one embodiment to receive a finger of the user, such as the forefinger or middle finger. To that end, an inner cylindrical surface of the ring 154 serves as the grip surface 118 against which the user's finger rests to enable the finger to handle and control movement of the ultrasound probe 40. It is thus noted that the finger-grip portion 110, i.e., the cylindrical ring 154 in the present embodiment, further serves as one example of a retention portion to retain engagement of the ultrasound probe 40 with the user's finger(s).

In one embodiment, the size of the cylindrical ring 154 can be configured such that the user's finger can be inserted varying distances through the ring in order to encounter a fit suitable for moving and handling the ultrasound probe 40. As such, the cylindrical ring 154 can be sized in one or more of varying diameters. In another embodiment, an insert can be removably fitted in to the cylindrical ring 154 to adjust the size of the ring opening to accommodate differently sized fingers. In yet another embodiment, the cylindrical ring 154 can have a varying diameter, such as being relatively wider at either end, so as to introduce freedom of movement for a finger inserted therein.

The ultrasound probe 40 of the embodiment of FIGS. 7A-7D further includes the stabilizing portion 146 configured to maintain the ultrasound probe in an upright, usable position on the skin surface of the patient even when the user's hand is not contacting the ultrasound probe. In the present embodiment, the stabilizing portion 146 includes the cylindrical ring 154 itself, best seen in FIG. 7D. The cylindrical ring 154 extends downward (from the perspective shown in FIG. 7D) so as to provide a lower surface substantially flush with the lens 108 of the ultrasound probe head 44. In this way, the bottom portion of the cylindrical ring 154 stabilizes the ultrasound probe 40 by providing a second contacting surface for the skin, in addition to the lens 108.

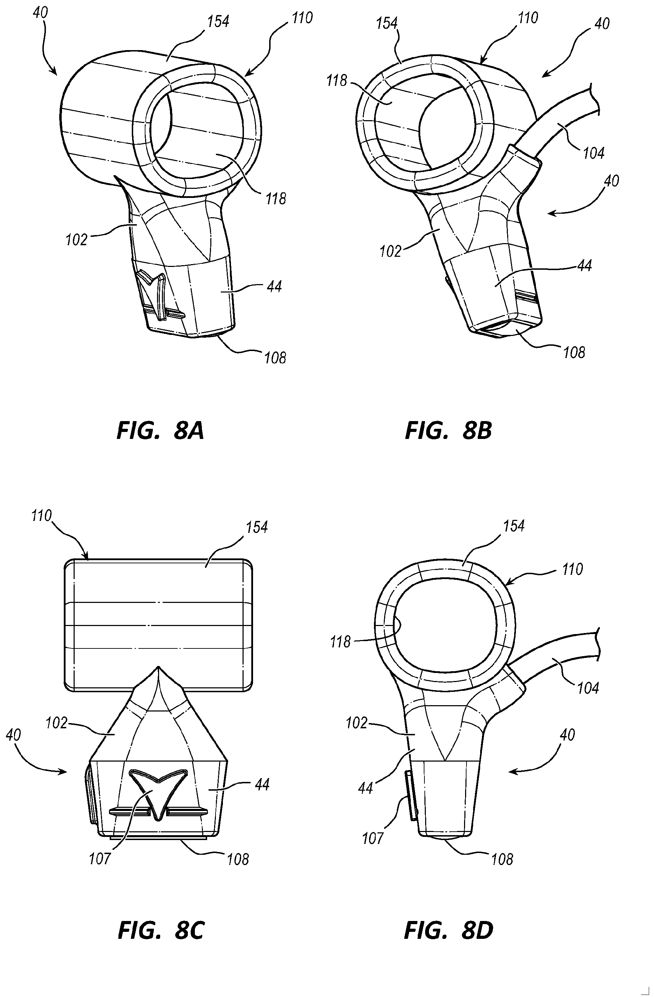

Reference is now made to FIGS. 8A-8D, which depict various details of the ultrasound probe 40 and its finger-grip portion 110 for enabling grasping, supporting, and handling of the ultrasound probe with one or more fingers of a single hand of the user while leaving one or more fingers of the user's hand free to be used in other ways, according to another embodiment.

As shown, the finger-grip portion 110 includes the cylindrical ring 124 extending from the body 102 of the ultrasound probe 40. The cylindrical ring 154 is sized in one embodiment to receive a finger of the user, such as the forefinger or middle finger. To that end, an inner cylindrical surface of the cylindrical ring 154 serves as the grip surface 118 against which the user's finger rests to enable the finger to handle and control movement of the ultrasound probe 40. It is thus noted that the finger-grip portion 110, i.e., the cylindrical ring 154 in the present embodiment, further serves as one example of a retention portion to retain engagement of the ultrasound probe 40 with the user's finger(s).

In one embodiment, the size of the cylindrical ring 154 can be configured such that the user's finger can be inserted varying distances through the ring in order to encounter a fit suitable for moving and handling the ultrasound probe 40. As such, the cylindrical ring 154 can be sized in one or more of varying diameters. In another embodiment, an insert can be removably fitted in to the cylindrical ring 154 to adjust the size of the ring opening to accommodate differently sized fingers.

The finger-grip portion 110, i.e., the cylindrical ring 154 in the embodiment of FIGS. 8A-8D, enables the user's finger to be positioned substantially co-linearly above and in a spaced-apart relationship with respect to the lens 108 of the ultrasound probe head 44. This enables the user to impart relatively fine amounts of pressure, or traction, to the skin surface via pressing down the ultrasound probe 40 thereon, and provides enhanced control over the ultrasound probe.

Note in the present embodiment that the cable 104, which operably connects the ultrasound probe 40 to the console 20 (FIG. 1), extends from the ultrasound-probe body 102 at an angle from the use position of the ultrasound probe (i.e., from the perspective shown in FIG. 8D). This cable configuration can reduce torque on the ultrasound probe 40 by the cable 104 as well as keep the cable clear of the user's hand. Such a configuration can be included in other of the embodiments described herein.

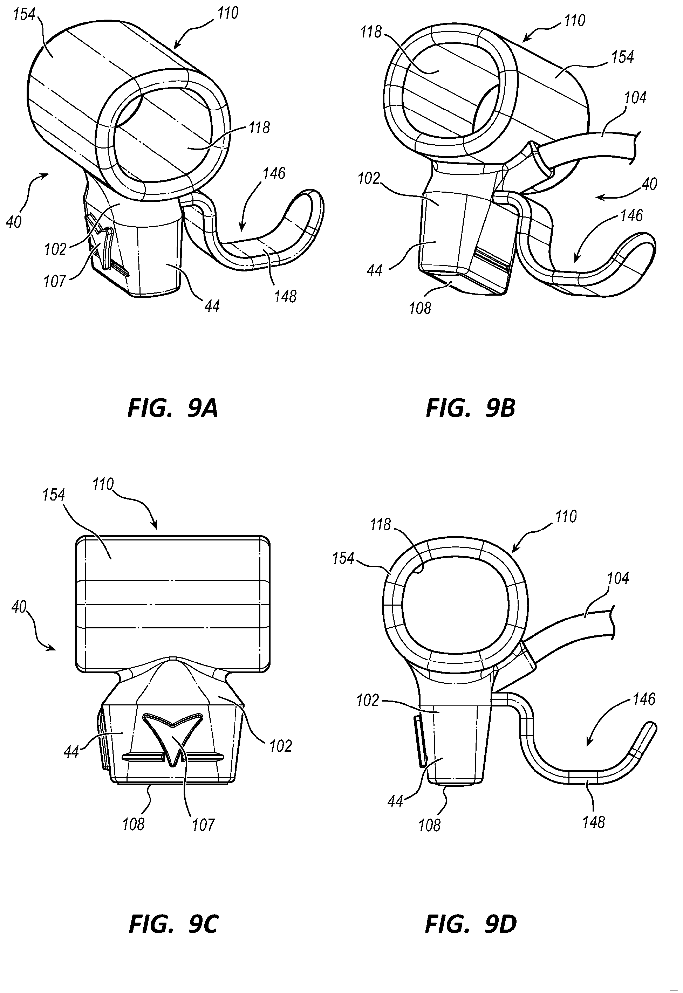

Reference is now made to FIGS. 9A-9D, which depict various details of the ultrasound probe 40 and its finger-grip portion 110 for enabling grasping, supporting, and handling of the ultrasound probe with one or more fingers of a single hand of the user while leaving one or more fingers of the user's hand free to be used in other ways, according to another embodiment.

As shown, the embodiment of FIGS. 9A-9D is similar to that shown in FIGS. 8A-8D, with the addition of the stabilizing portion 146- here, the angled leg 148 similar to that shown in the embodiment of FIGS. 6A-6E. Note that the particular shape of the angled leg 148 of FIGS. 9A-9D differs in shape somewhat from the angled leg of FIGS. 6A-6E, while still being configured to maintain the ultrasound probe in an upright, usable position on the skin surface of the patient even when the user's hand is not contacting the ultrasound probe. As before, the angled leg 148 extends downward (from the perspective shown in FIG. 9D) so as to provide a lower surface substantially flush with the lens 108 of the ultrasound-probe head 44. In this way, the leg 148 stabilizes the ultrasound probe 40 by providing a second contacting surface for the skin, in addition to the lens 108.

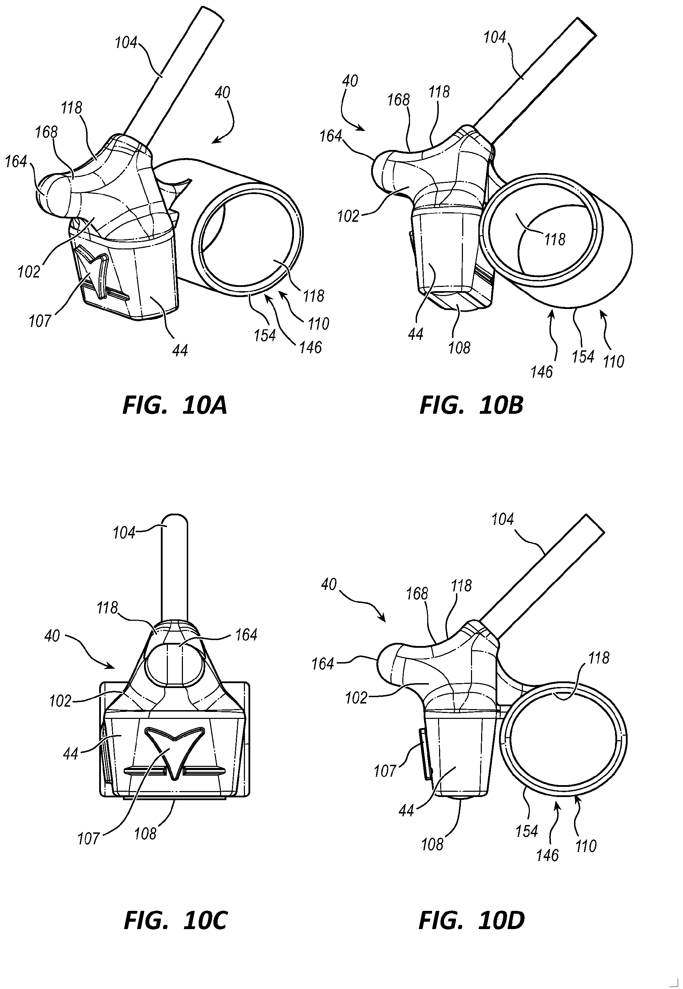

Reference is now made to FIGS. 10A-10D, which depict various details of the ultrasound probe 40 and its finger-grip portion 110 for enabling grasping, supporting, and handling of the ultrasound probe with one or more fingers of a single hand of the user while leaving one or more fingers of the user's hand free to be used in other ways, according to another embodiment. As shown, the finger-grip portion 110 includes a cylindrical ring 154 extending from the main body 102 of the ultrasound probe 40. The cylindrical ring 154 is sized in one embodiment to receive a first finger of the user, such as the middle finger. To that end, an inner cylindrical surface of the ring 154 serves as the grip surface 118 against which the user's finger rests to enable the finger to handle and control movement of the ultrasound probe 40. It is thus noted that the finger-grip portion 110, i.e., the cylindrical ring 154 in the present embodiment, further serves as one example of a retention portion to retain engagement of the ultrasound probe 40 with the user's finger(s).

In one embodiment, the size of the cylindrical ring 154 can be configured such that the user's finger can be inserted varying distances through the ring in order to encounter a fit suitable for moving and handling the ultrasound probe 40. As such, the cylindrical ring 154 can be sized in one or more of varying diameters. In another embodiment, an insert can be removably fitted in to the cylindrical ring 154 to adjust the size of the ring opening to accommodate differently sized fingers.

The ultrasound-probe body 102 further defines a protrusion 164 and a saddle 168 to provide a second grip surface 118 on which a second finger of the user's hand, such as the forefinger, can rest to further support and handle the ultrasound probe 40. Thus, two fingers of a user's single hand can be employed in the present embodiment to support and use the ultrasound probe 40. The saddle 168 enables the user's finger to be positioned substantially co-linearly above and in a spaced-apart relationship with respect to the lens 108 of the ultrasound-probe head 44. This enables the user to impart relatively fine amounts of pressure, or traction, to the skin surface via pressing down the ultrasound probe 40 thereon, and provides enhanced control over the ultrasound probe.

The ultrasound probe 40 of the embodiment of FIGS. 10A-10D further includes the stabilizing portion 146 configured to maintain the ultrasound probe in an upright, usable position on the skin surface of the patient even when the user's hand is not contacting the ultrasound probe. In the present embodiment, the stabilizing portion 146 includes the cylindrical ring 154 itself, best seen in FIG. 10D. The cylindrical ring 154 extends downward (from the perspective shown in FIG. 10D) so as to provide a lower surface substantially flush with the lens 108 of the ultrasound-probe head 44. In this way, the bottom portion of the cylindrical ring 154 stabilizes the ultrasound probe 40 by providing a second contacting surface for the skin, in addition to the lens 108.

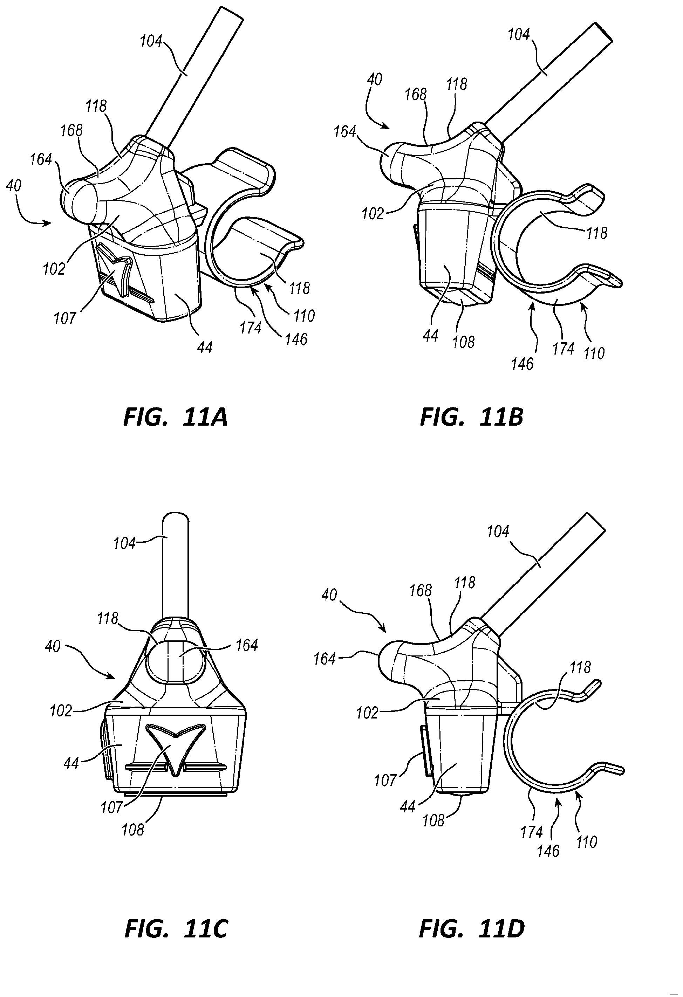

Reference is now made to FIGS. 11A-11D, which depict various details of the ultrasound probe 40 and its finger-grip portion 110 for enabling grasping, supporting, and handling of the ultrasound probe with one or more fingers of a single hand of the user while leaving one or more fingers of the user's hand free to be used in other ways, according to another embodiment.

As shown, the embodiment of FIGS. 11A-11D is similar to that shown in FIGS. 10A-10D, with the finger-grip portion 110 including a cylindrical hook 174 extending from the ultrasound-probe body 102. The cylindrical hook 174 is approximately C-shaped and is sized in one embodiment to receive a finger of the user, such as the forefinger or middle finger, while enabling an opening for ease of finger insertion/removal. An inner surface of the hook 134 serves as the grip surface 118 against which the user's finger rests to enable the finger to handle and control movement of the ultrasound probe 40. In one embodiment, the cylindrical hook 174 is resilient such that it can deform a limited amount to conform to the size of the user's finger and remain connected therewith. It is thus noted that the finger-grip portion 110, i.e., the hook 134 in the present embodiment, further serves as one example of a retention portion to retain engagement of the ultrasound probe 40 with the user's finger(s).

Note that in one embodiment the cylindrical hook 174 can include an oval cross-sectional shape to further enhance its engagement with the user's finger. In one embodiment, excess material comprising a sterile cover that is draped over the ultrasound probe 40 can be inserted into the interior portion of the cylindrical hook 174 in order to increases the engagement of the cylindrical hook with the user's finger (and note that this technique can be employed in connection with the other embodiments herein, including those depicted in FIGS. 4A, 5A, 7A, 8A, 9A, 10A, and 12A. In addition, the cylindrical hook 174 in one embodiment can be removable as to enable cylindrical rings or other finger retention portions of different sizes/configurations to be interchanged on the ultrasound probe 40.

Also, as with the cylindrical ring 154 of the embodiment shown in FIGS. 10A-10D, the cylindrical hook 174 of the present embodiment serves as the stabilizing portion 146, configured to maintain the ultrasound probe 40 in an upright, usable position on the skin surface of the patient even when the user's hand is not contacting the ultrasound probe.

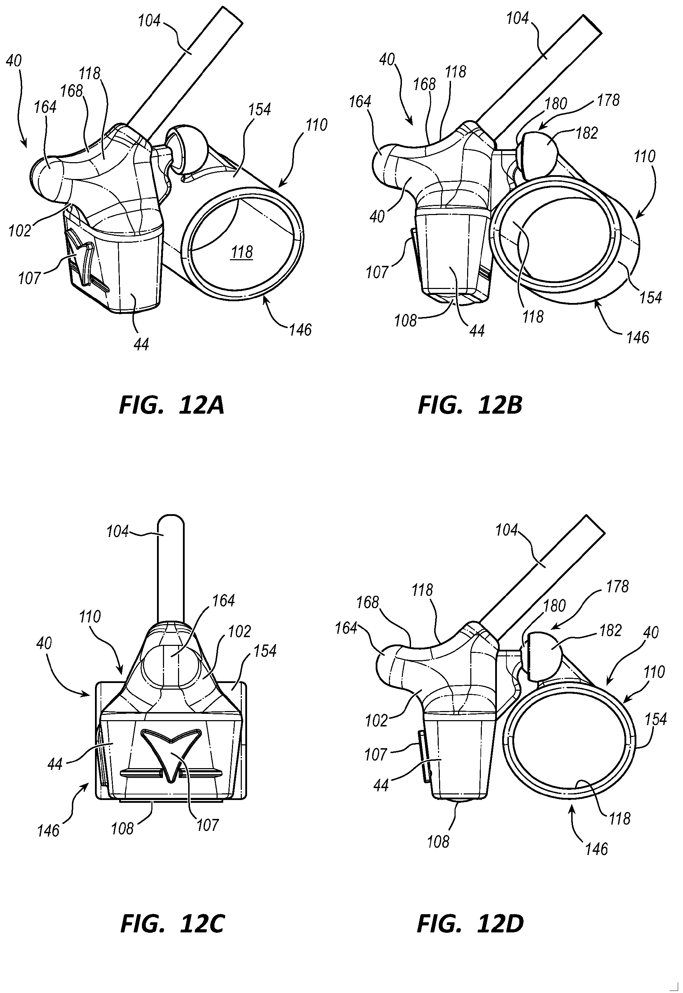

Reference is now made to FIGS. 12A-12D, which depict various details of the ultrasound probe 40 and its finger-grip portion 110 for enabling grasping, supporting, and handling of the ultrasound probe with one or more fingers of a single hand of the user while leaving one or more fingers of the user's hand free to be used in other ways, according to another embodiment.

As shown, the embodiment of FIGS. 12A-12D is similar to that shown in FIGS. 10A-10D, with the finger-grip portion 110 including the cylindrical ring 154, which further serves as the stabilizing portion 146. The embodiment shown in FIGS. 12A-12D includes an articulating component 178 disposed between the cylindrical ring 154 and the main portion of the ultrasound-probe body 102. In detail, the articulating component 178 includes a ball 180 included on the main portion of the ultrasound-probe body 102 and a corresponding socket 182 included on the cylindrical ring and sized to receive the ball therein so as to enable articulating movement between the ultrasound-probe head 44 and the cylindrical ring 154 through which a user's finger is received during an ultrasound imaging procedure. The articulating component 178 enables relative movement between the ultrasound-probe head 44 and the cylindrical ring, thus enabling freedom of movement of the ultrasound probe 40. This is useful when it is desired to lift the lens 108 above the skin surface while desiring to keep one or more fingers on the skin surface. Note that other articulating and jointed structures can be employed to provide such relative movement, such as a hinge in one embodiment. Also, the ball and socket configuration can be reversed in position, in one embodiment.

Note that, in the present and previous embodiments discussed above, the ultrasound-probe head 44, lens 108, and orientation arrow 107 are configured similar to standard ultrasound probe heads so as to lend familiarity to the user in terms of placement of the ultrasound-probe head and lens on the skin surface and inserting needles, etc.

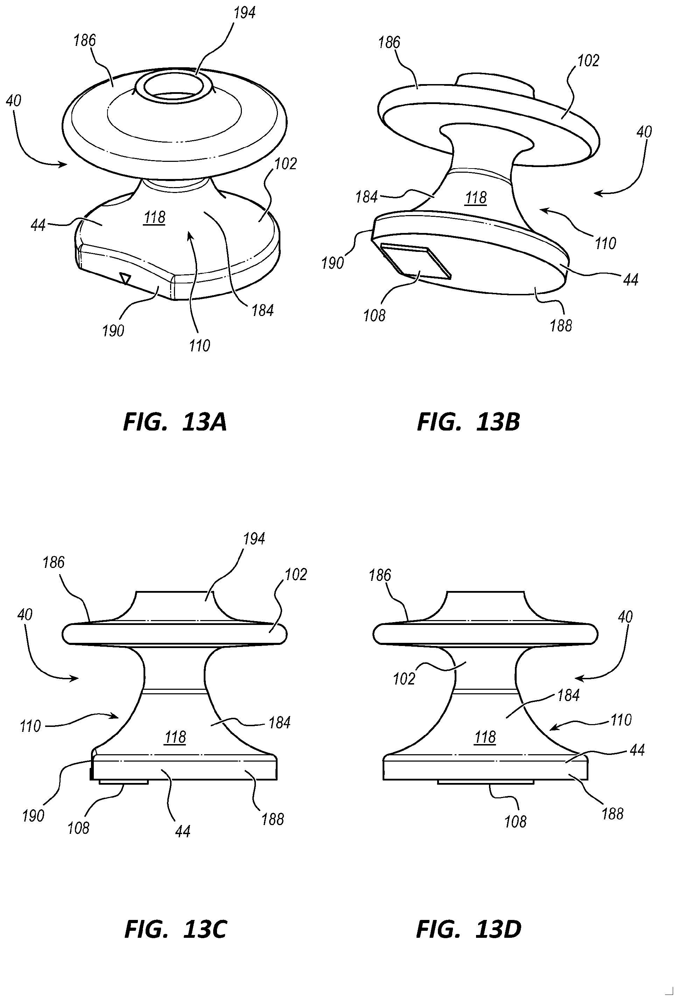

Reference is now made to FIGS. 13A-13D, which depict various details of the ultrasound probe 40 and its finger-grip portion 110 for enabling grasping, supporting, and handling of the ultrasound probe with one or more fingers of a single hand of the user while leaving one or more fingers of the user's hand free to be used in other ways, according to another embodiment. As shown, the ultrasound-probe body 102 includes a top disk 186 and a bottom disk 188 that are separated by the finger-grip portion 110, here configured as an hourglass-shaped surface 184 that defines the grip surface 118. Two fingers of a user's single hand, such as the forefinger and middle finger (though other digits could be used), can straddle the hourglass-shaped surface 184 to grasp and handle the ultrasound probe 40. A flat side surface 190 is included on the bottom disk 188 proximate the lens 108, which is disposed on the bottom of the ultrasound-probe head 44, from the perspective shown in FIGS. 13C and 13D. A cable can extend from a cable conduit 194 at a top surface of the ultrasound-probe body 102. As in previous embodiments, the ultrasound-probe body design shown in the present embodiment enables a finger to be positioned substantially directly over the lens 108 in a spaced-apart arrangement. Note that the flat bottom surface of the bottom disk 188, best seen in FIG. 13B, serves as a stabilizing portion in the present embodiment for enabling the ultrasound probe 40 to rest without assistance on the skin surface of the patient.

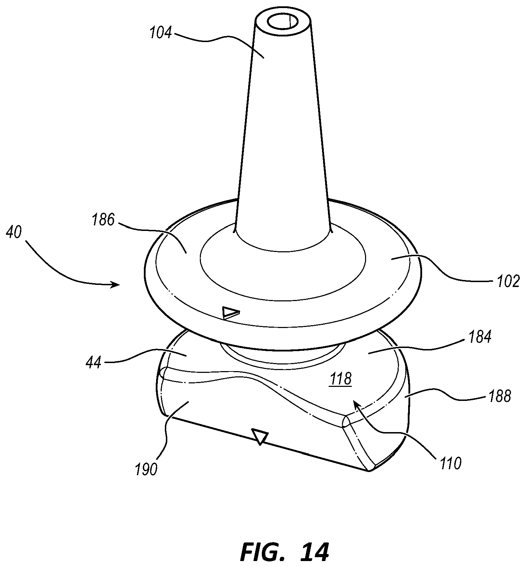

FIG. 14 depicts the ultrasound probe 40 according to one embodiment. The ultrasound probe 40 here is similar to that shown in the embodiment of FIGS. 13A-13D, but with the flat side surface 190, proximate the lens 108, being relatively larger.

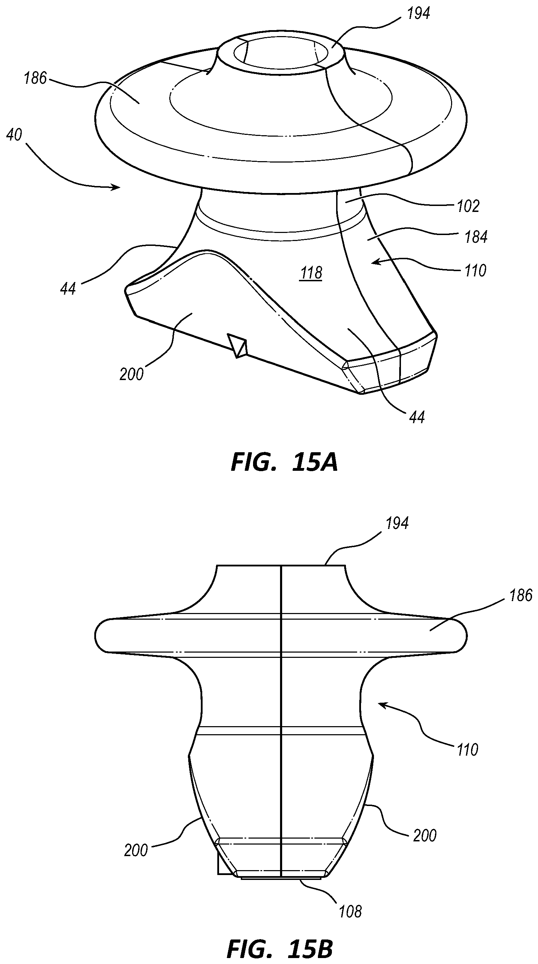

FIGS. 15A and 15B depict yet another variation of the embodiment shown in FIGS. 13A-13D, wherein the bottom disk of the ultrasound-probe body 102 is omitted and two chamfered surfaces 200 are defined on a bottom portion of the ultrasound-probe body proximate the lens 108. These and other variations to the ultrasound-probe body 102 while still enabling grasping and handling of the ultrasound probe 40 with one or more fingers but while still enabling other fingers of the user's hand to be free for other purposes, are therefore contemplated.

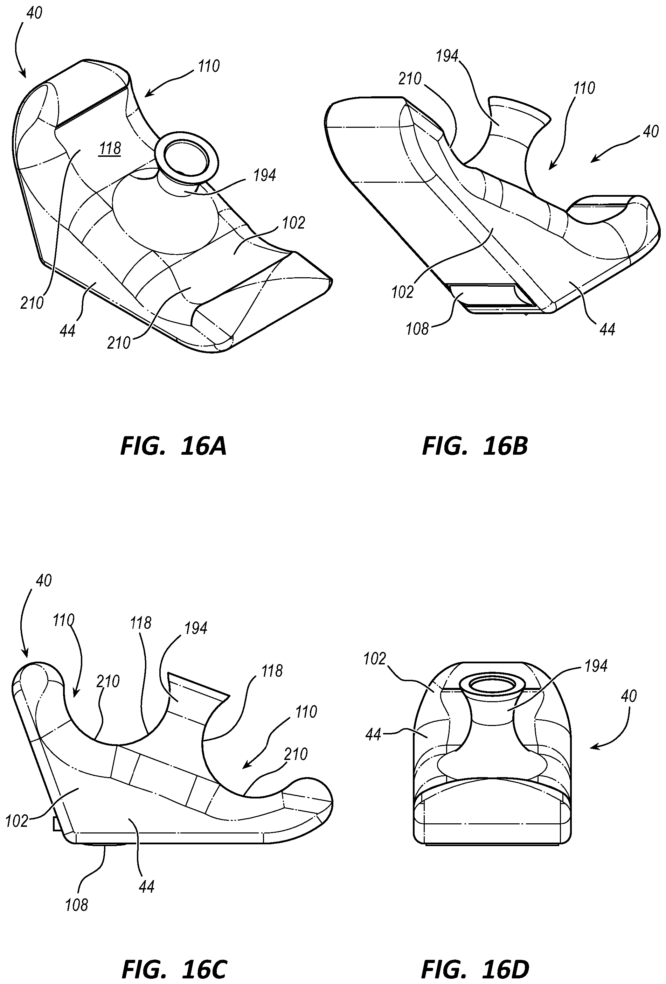

FIGS. 16A-16D depict various details of the ultrasound probe 40 and its finger-grip portion 110 for enabling grasping, supporting, and handling of the ultrasound probe with one or more fingers of a single hand of the user while leaving one or more fingers of the user's hand free to be used in other ways, according to another embodiment. As shown, the ultrasound-probe body 102 includes a generally wedge-shaped configuration, with the lens 108 of the ultrasound-probe head 44 disposed on a bottom surface thereof, from the perspective shown in FIGS. 16C and 16D. Note that the flat bottom surface of the ultrasound-probe body 102, best seen in FIG. 16B, serves as a stabilizing portion in the present embodiment for enabling the ultrasound probe 40 to rest without assistance on the skin surface of the patient.

The cable conduit 194 extends from the ultrasound-probe body 102 and two finger-grip portions 110 are included, namely, two channels 210 that are defined by the body and are disposed adjacent the cable conduit so as to provide two concavely shaped grip surfaces 118. Two fingers of a single hand of the user, such as the forefinger and middle finger, can be received into the channel 210 to support and handle the ultrasound probe 40, while enabling other fingers of the user's hand to be used for other purposes. These and other ultrasound probe body shapes and finger-grip portion configurations are therefore contemplated.

Note that the ultrasound probes discussed herein are further configured to enable a sterile cover to be thereover and for elastic bands to be used to secure the cover to the ultrasound probe. The ultrasound probes herein are also configured to be symmetrical along at least one midline axis so as to enable both left-handed and right-handed use.

In one embodiment, it is appreciated that icons/symbols may be placed on the ultrasound probe body 102 to assist a user in knowing where to place finger(s) for use of the ultrasound probe 40.

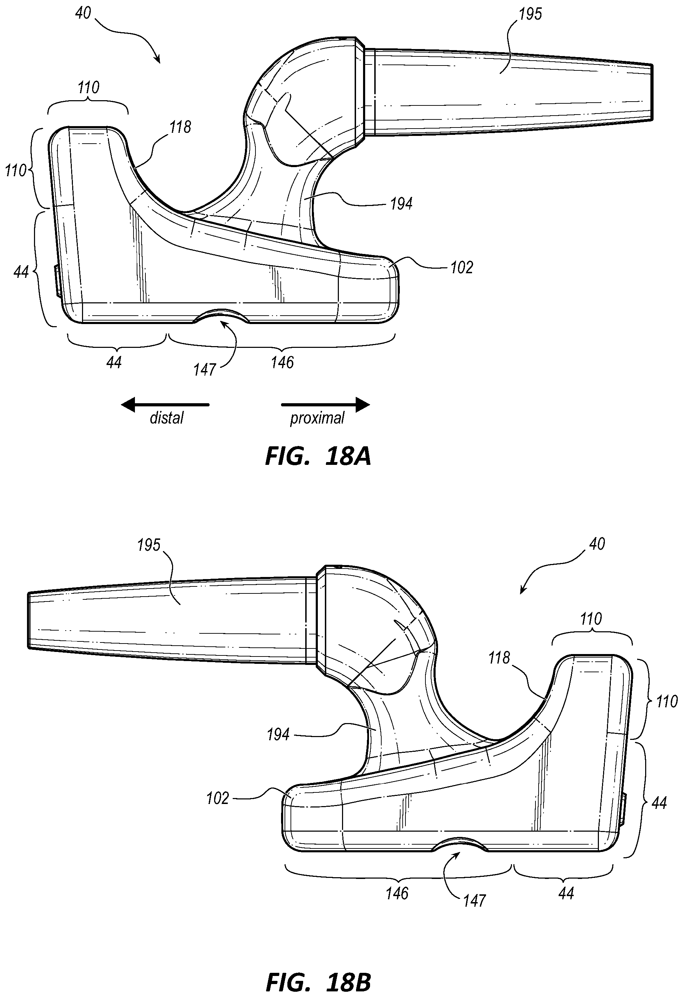

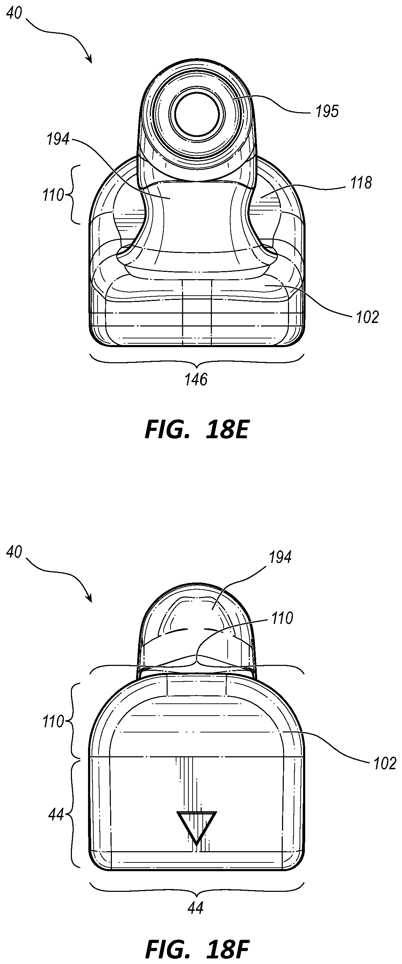

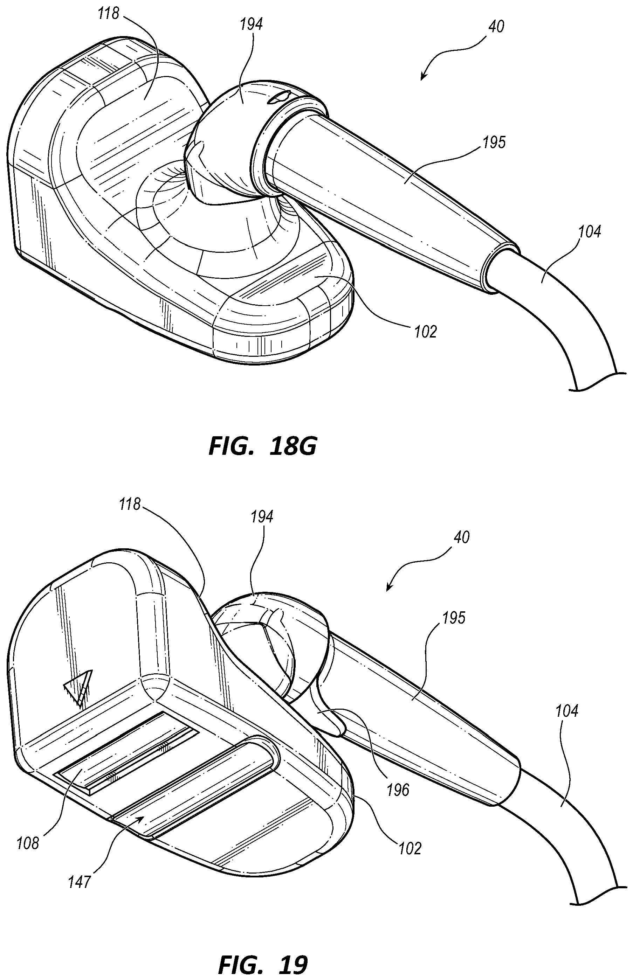

FIG. 18A-18G provide various views of an ultrasound probe 40 in accordance with some embodiments. FIG. 19 provides a perspective view of an ultrasound probe 40 in accordance with some embodiments. The ultrasound probe 40 of FIG. 19 shares the features of the ultrasound probe 40 of FIGS. 18A-18G set forth below; however, the ultrasound probe 40 of FIG. 19 includes an added feature, namely a conduit-tube extension.

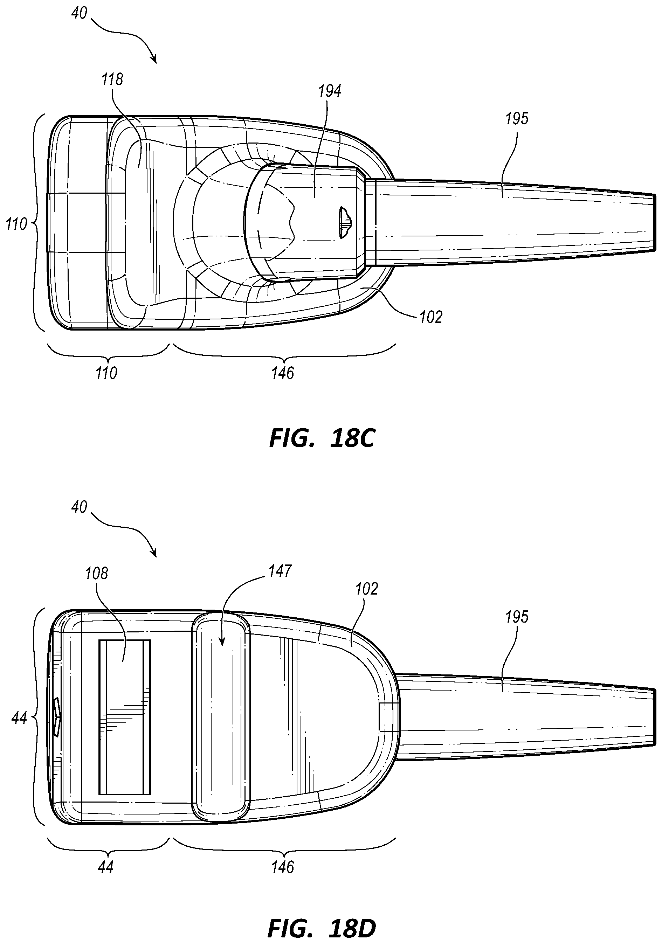

As shown, the ultrasound probe 40 includes a body 102, a lens 108, a stabilizing portion 146 of the body 102, and a finger-grip portion 110 of the body 102. As best seen in FIGS. 18C-18F, the ultrasound probe 40 is bilaterally symmetrical about a plane of symmetry of the ultrasound probe 40.

The body 102 of the ultrasound probe 40 can be formed of a molded thermoplastic such as polyphenylsulfone. Indeed, at least a top portion of the body 102 shown in FIG. 18C can be formed of molded thermoplastic halves coupled together along the plane of symmetry of the ultrasound probe 40. A bottom portion of the body 102 shown in FIG. 18D can be formed of a molded thermoplastic coupled to the top portion of the body 102. The bottom portion of the body 102 can include a cutout or molded latitudinal hole configured to hold the lens 108.

A head portion 44 of the ultrasound probe 40 is designated by at least the lens 108 disposed in the cutout or molded latitudinal hole of the body 102. The lens 108 is configured to pass ultrasound signals therethrough. The ultrasound signals include emitted ultrasound signals emitted by an array of piezoelectric transducers within the ultrasound probe 40 behind the lens 108. The ultrasound signals also include reflected ultrasound signals reflected into the ultrasound probe 40 such as from a tissue being imaged.

The stabilizing portion 146 of the ultrasound probe 40 is composed of a longitudinal extension of the body 102 extending away from the head portion 44 of the ultrasound probe 40. The stabilizing portion 146 is configured to stabilize the ultrasound probe 40 on a skin surface of a patient without assistance from a user of the ultrasound probe 40.

The stabilizing portion 146 of the ultrasound probe 40 includes a latitudinal channel 147 extending from side to side of two longitudinal sides of the ultrasound probe 40. The latitudinal channel 147 reduces a surface area of a generally flat surface of the stabilizing 146 portion intended to face the skin surface of the patient. The reduced surface area of the stabilizing portion 146 facilitates movement of the ultrasound probe 40 across the skin surface of the patient by reducing contact area between the surface of the stabilizing portion 146 and the skin surface of the patient. The reduced surface area of the stabilizing portion 146 also reduces suction between the surface of the stabilizing portion 146 and the skin surface of the patient when gel is therebetween and the ultrasound probe 40 is removed from the patient. In addition, the latitudinal channel 147 provides a space between the ultrasound probe 40 or the stabilizing portion 146 thereof and the skin surface of the patient into which a finger can be inserted for lifting the ultrasound probe 40 from the skin surface of the patient when removing the ultrasound probe 40 therefrom.

The finger-grip portion 110 of the ultrasound probe 40 is composed of a generally transverse extension of the body 102 extending away from the head portion 44 of the ultrasound probe 40. The finger-grip portion 110 is configured to enable the user to grasp and maneuver the ultrasound probe 40 during use thereof with no more than two or three fingers of a single hand. For example, the two fingers can be a thumb and an index finger. The finger-grip portion 110 of the ultrasound probe includes a concave grip surface 118 opposite a generally flat surface. The grip surface 118 is an extension of a concave surface of the stabilizing portion 146 of the ultrasound probe 40 intended to face away from the skin surface of the patient. In other words, the concave surface of the stabilizing portion 146 is opposite the generally flat surface of the stabilizing portion 146 intended to face the skin surface of the patient.

The ultrasound probe 40 includes a cable conduit 194 composed of a curved extension of the body 102 of the ultrasound probe 40 extending away from both the stabilizing and head portions of the ultrasound probe 40. The cable conduit 194 is configured to house a distal-end portion of a power-and-data cable such as the cable 104 for connecting the ultrasound probe 40 to the console 20. The cable conduit 194 is also configured to keep the power-and-data cable away from the head portion 44 of the ultrasound probe 40, thereby reducing interference by the power-and-data cable when using the ultrasound probe 40. As shown in FIG. 19, the cable conduit 194 can include an extension 196 on a side of the cable conduit 194 intended to face the patient. The extension 196 is configured to reinforce the cable boot 195 set forth below in keeping the power-and-data cable farther away from the head portion 44.

The ultrasound probe 40 includes a cable boot 195 having a distal-end portion disposed in an opening of the cable conduit 194. The cable boot 195 is configured to house the distal-end portion of the power-and-data cable proximal of the cable conduit 194. The cable boot 195 is also configured to keep the power-and-data cable farther away from the head portion 44 of the ultrasound probe 40. The cable boot 195 is also configured to attenuate a bending radius of the power-and-data cable about the opening of the cable conduit 194. Attenuating the bending radius of the power-and-data cable reduces a risk of damaging the power-and-data cable at the opening of the cable conduit 194 where the power-and-data cable is most likely to overbend from moving the ultrasound probe 40 when using the ultrasound probe 40.

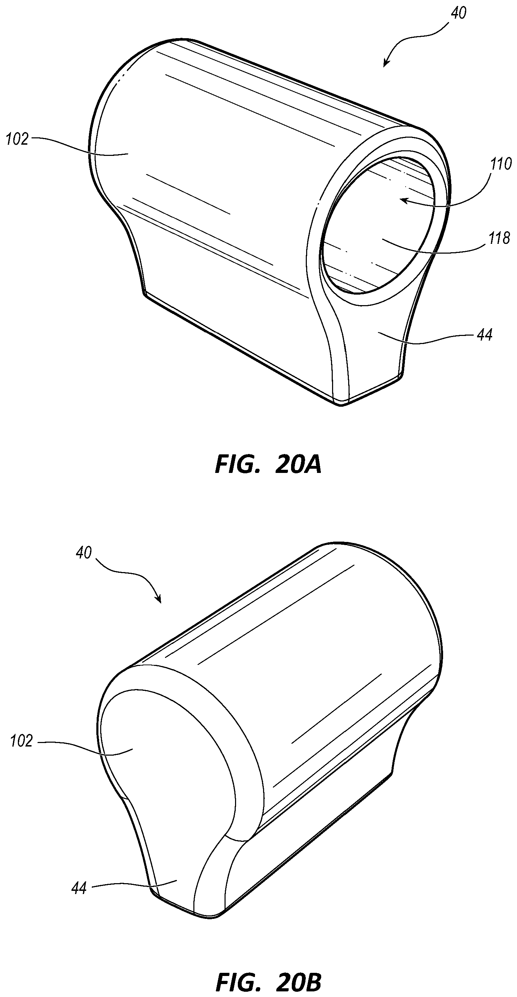

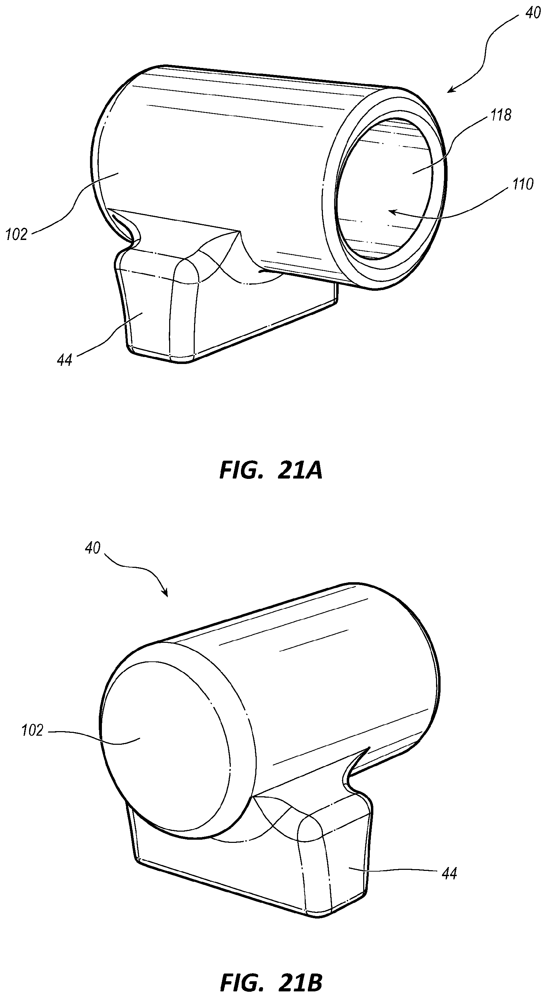

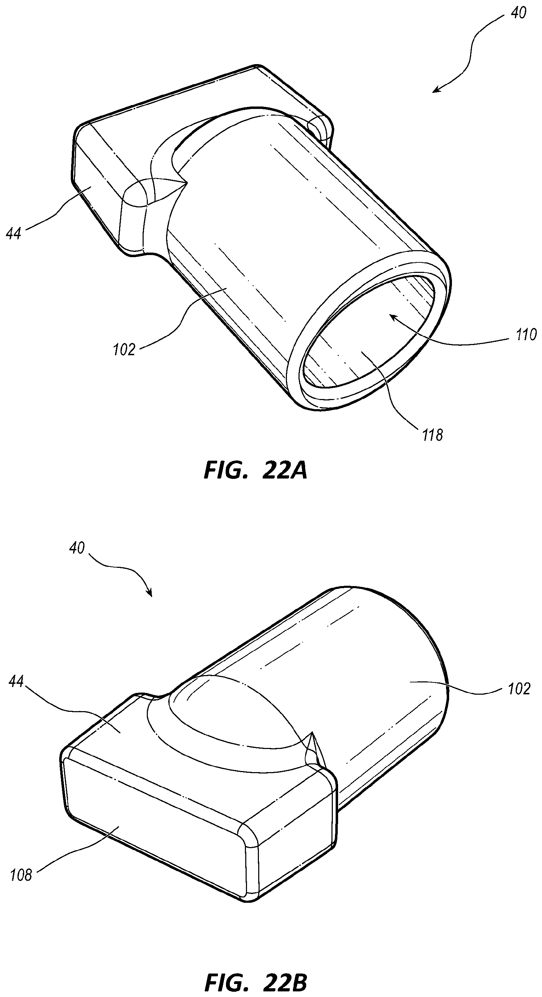

FIGS. 20A and 20B, FIGS. 21A and 21B, FIGS. 22A and 22B, FIGS. 23A and 23B provide different views of different ultrasound probes in accordance with some embodiments.

As shown, each ultrasound probe 40 includes a body 102, a head portion 44 of the body 102, and a finger-grip portion 110 of the body 102. Each ultrasound probe 40 is bilaterally symmetrical about a plane of symmetry of the ultrasound probe 40.

The body 102 of each ultrasound probe 40 can be formed of a molded thermoplastic such as polyphenylsulfone.

The head portion 44 of each ultrasound probe 40 is designated by at least a lens 108 disposed in a cutout or molded hole of the body 102. The lens 108 is configured to pass emitted and reflected ultrasound signals therethrough.

The finger-grip portion 110 of each ultrasound probe 40 is composed of a thimble-type structure of the body 102 of the ultrasound probe 40. The finger-grip portion 110 is configured to enable a user to insert, for example, an index or middle finger therein for maneuvering the ultrasound probe 40 during use thereof. The finger-grip portion 110 includes a grip surface 118 as set forth above.

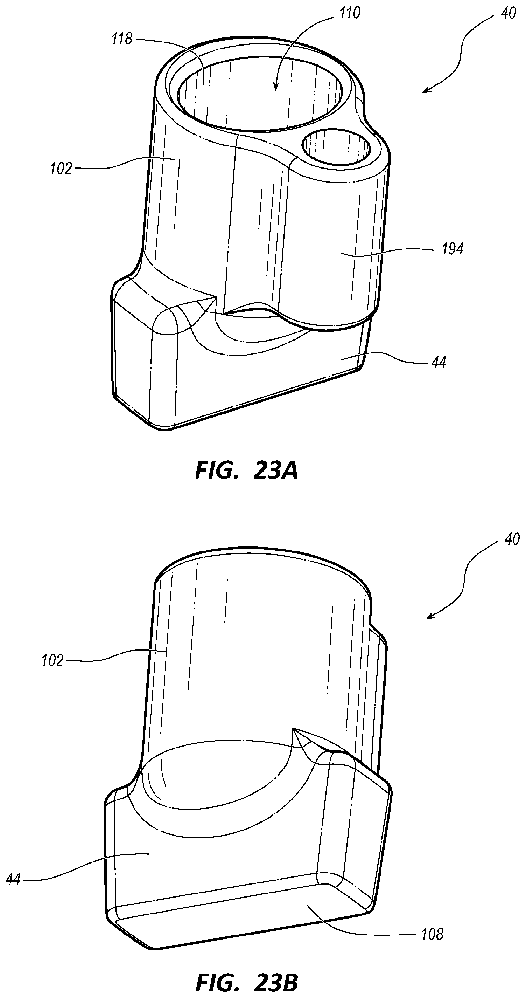

At least the ultrasound probe 40 of FIGS. 23A and 23B is shown with a cable conduit 194, which cable conduit 194 is configured to house a distal-end portion of a power-and-data cable such as the cable 104 for connecting the ultrasound probe 40 to the console 20. While the cable conduit 194 is only shown for the ultrasound probe FIGS. 23A and 23B, it should be understood that any ultrasound probe of the ultrasound probes in FIGS. 20A and 20B, FIGS. 21A and 21B, and FIGS. 22A and 22B can include such a cable conduit.

### Methods

A method of the ultrasound probe 40 includes an obtaining step of obtaining the ultrasound probe 40. For example, the obtaining step can include obtaining the ultrasound probe 40 of FIGS. 18A-18G, which includes the body 102, the stabilizing portion 146 of the body 102, and the finger-grip portion 110 of the body 102. Again, the body 102 of the foregoing ultrasound probe 40 has the lens 108 disposed in the cutout or molded latitudinal hole of the body 102, which designates the head portion 44 of the ultrasound probe 40. The stabilizing portion 146 is composed of the longitudinal extension of the body 102 extending away from the head portion 44 of the ultrasound probe 40. The finger-grip portion 110 is composed of the generally transverse extension of the body 102 extending away from the head portion 44 of the ultrasound probe 40.

The method also includes a connecting step of connecting the power-and-data cable of the ultrasound probe 40 to the console 20 of the ultrasound imaging system 10. The cable 104 extends from the cable conduit 194 and the cable boot 195 of the ultrasound probe 40. Again, the cable conduit 194 and the cable boot 195 is configured to keep the power-and-data cable away from the head portion 44 of the ultrasound probe 40 when using the ultrasound probe 40.

The method can include an applying step of applying an ultrasound gel to a skin surface of a patient before placing the ultrasound probe 40 on the skin surface of the patient.

The method also includes a grasping step of grasping the ultrasound probe 40. The grasping step includes placing at least one of an index finger or a middle finger on the concave grip surface 118 of the finger-grip portion 110 of the ultrasound probe 40, as well as a thumb on the opposing, generally flat surface of the finger-grip portion 110. The grasping step orients a palm of the single hand to which the foregoing fingers belong substantially parallel to the skin surface of the patient.

The method also includes a placing step of placing the head and stabilizing portions of the ultrasound probe 40 on the skin surface of the patient.

The method also includes an enabling step of enabling probing ultrasound signals to be emitted into the patient from the array of piezoelectric transducers within the ultrasound probe 40 for ultrasound imaging. The method also includes another enabling step of enabling reflected ultrasound signals to be reflected from the patient into the array of piezoelectric transducers within the ultrasound probe 40 for conversion into electrical signals for corresponding ultrasound images. The enabling of the foregoing enabling steps can include turning on the console 20, selecting one or more imaging modes of the ultrasound imaging system 10, or initiating ultrasound imaging with the ultrasound probe 40 by activating a start button or the like on the console 20.

The method also includes a moving step of moving the ultrasound probe 40 for the ultrasound imaging by the finger-grip portion 110 of the ultrasound probe 40 using no more than the foregoing two or three fingers of the single hand. The moving step does not overbend the power-and-data cable due to attenuation of the bending radius of the power-and-data cable by the cable boot 195.

The method also includes a removing step of removing the ultrasound probe 40 from the skin surface of the patient. Again, the stabilizing portion 146 of the ultrasound probe 40 includes the latitudinal channel 147 extending from side to side of the two longitudinal sides of the ultrasound probe 40. The latitudinal channel 147 reduces the surface area of the generally flat surface of the stabilizing portion 146, thereby reducing gel-induced suction between the surface of the stabilizing portion 146 and the skin surface of the patient during the removing step. In addition, the latitudinal channel 147 provides a space between the ultrasound probe 40 and the skin surface of the patient into which, for example, the index finger can be inserted for lifting the ultrasound probe 40 when removing the ultrasound probe 40 from the skin surface of the patient.

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the concepts provided herein.

The following clauses relate to aspects of the invention that may or may not presently be claimed:
An ultrasound probe, comprising:
   a body;
   a lens disposed in a cutout of the body designating a head portion of the ultrasound probe, the lens configured to pass ultrasound signals therethrough;
   a stabilizing portion composed of a longitudinal extension of the body extending away from the head portion of the ultrasound probe, the stabilizing portion of the ultrasound probe configured to stabilize the ultrasound probe on a skin surface of a patient without assistance from a user of the ultrasound probe; and
   a finger-grip portion composed of a generally transverse extension of the body extending away from the head portion of the ultrasound probe, the finger-grip portion of the ultrasound probe configured to enable the user to grasp and maneuver the ultrasound probe during use thereof with no more than two or three fingers of a single hand.
2. The ultrasound probe of clause 1, further comprising a cable conduit composed of a curved extension of the body extending away from both the stabilizing and head portions of the ultrasound probe, the cable conduit configured to house a distal-end portion of a power-and-data cable for connecting the ultrasound probe to a console and keep the power-and-data cable away from the head portion of the ultrasound probe.
3. The ultrasound probe of clause 2, further comprising a cable boot having a distal-end portion disposed in an opening of the cable conduit, the cable boot configured to house the distal-end portion of the power-and-data cable proximal of the cable conduit and keep the power-and-data cable farther away from the head portion of the ultrasound probe.
4. The ultrasound probe of clause 3, wherein the cable boot is configured to attenuate a bending radius of the power-and-data cable about the opening of the cable conduit, thereby reducing a risk of damaging the power-and-data cable at the opening of the cable conduit where the power-and-data cable is most likely to overbend.
5. The ultrasound probe of any clause of clauses 1-4, wherein the stabilizing portion of the ultrasound probe includes a latitudinal channel extending from side to side of two longitudinal sides of the ultrasound probe, thereby reducing a surface area of a generally flat surface of the stabilizing portion intended to face the skin surface of the patient.
6. The ultrasound probe of clause 5, wherein the surface area of the stabilizing portion of the ultrasound probe reduced by the latitudinal channel facilitates movement of the ultrasound probe across the skin surface of the patient by reducing contact area between the surface of the stabilizing portion and the skin surface of the patient.
7. The ultrasound probe of clause 5 or 6, wherein the surface area of the stabilizing portion of the ultrasound probe reduced by the latitudinal channel reduces suction between the surface of the stabilizing portion and the skin surface of the patient when gel is therebetween and the ultrasound probe is removed from the patient.
8. The ultrasound probe of any clause of clauses 1-7, wherein the finger-grip portion of the ultrasound probe includes a concave surface opposite a generally flat surface.
9. The ultrasound probe of clause 8, wherein the concave surface of the finger-grip portion is an extension of a concave surface of the stabilizing portion of the ultrasound probe intended to face away from the skin surface of the patient.
10. The ultrasound probe of any clause of clauses 1-9, wherein the ultrasound probe is bilaterally symmetrical about a plane of symmetry of the ultrasound probe.
11. The ultrasound probe of clause 10, wherein at least a portion of the body is molded thermoplastic coupled together along the plane of symmetry of the ultrasound probe.
12. An ultrasound system, comprising:
   a console configured for processing and depicting ultrasound images on a display of the console; and
   an ultrasound probe including
      a body;
      a lens disposed in a cutout of the body designating a head portion of the ultrasound probe, the lens configured to pass ultrasound signals therethrough;
      a stabilizing portion composed of a longitudinal extension of the body extending away from the head portion of the ultrasound probe, the stabilizing portion of the ultrasound probe configured to stabilize the ultrasound probe on a skin surface of a patient without assistance from a user of the ultrasound probe;
      a cable conduit composed of a curved extension of the body extending away from both the stabilizing and head portions of the ultrasound probe, the cable conduit configured to house a distal-end portion of a power-and-data cable for connecting the ultrasound probe to the console; and
      a finger-grip portion composed of a generally transverse extension of the body extending away from the head portion of the ultrasound probe, the finger-grip portion of the ultrasound probe configured to enable the user to grasp and maneuver the ultrasound probe during use thereof with no more than two or three fingers of a single hand.
13. A method of an ultrasound probe, comprising:
   obtaining the ultrasound probe, the ultrasound probe including:
      a body having a lens disposed in a cutout of the body designating a head portion of the ultrasound probe,
      a stabilizing portion composed of a longitudinal extension of the body extending away from the head portion of the ultrasound probe, and
      a finger-grip portion composed of a generally transverse extension of the body extending away from the head portion of the ultrasound probe;
   placing the head and stabilizing portions of the ultrasound probe on a skin surface of a patient;
   enabling probing ultrasound signals to be emitted into the patient from an array of piezoelectric transducers within the ultrasound probe for ultrasound imaging; and
   moving the ultrasound probe for the ultrasound imaging by the finger-grip portion of the ultrasound probe using no more than two or three fingers of a single hand.
14. The method of clause 13, further comprising grasping the ultrasound probe before moving the ultrasound probe, the grasping including placing at least one of an index finger or a middle finger on a concave surface of the finger-grip portion of the ultrasound probe and a thumb on an opposing, generally flat surface of the finger-grip portion of the ultrasound probe.
15. The method of clause 14, wherein grasping the ultrasound probe orients a palm of the single hand substantially parallel to the skin surface of the patient.
16. The method of any clause of clauses 13-15, further comprising applying an ultrasound gel to the skin surface of the patient before placing the head and stabilizing portions of the ultrasound probe on a skin surface of a patient.
17. The method of clause 16, further comprising removing the ultrasound probe from the skin surface of a patient, the stabilizing portion of the ultrasound probe including a latitudinal channel extending from side to side of two longitudinal sides of the ultrasound probe that reduces a surface area of a generally flat surface of the stabilizing portion, thereby reducing gel-induced suction between the surface of the stabilizing portion and the skin surface of the patient when removing the ultrasound probe from the skin surface of the patient.
18. The method of any clause of clauses 13-17, further comprising connecting a power-and-data cable of the ultrasound probe to a console of an ultrasound imaging system, the cable extending from a cable conduit of the ultrasound probe composed of a curved extension of the body extending away from both the stabilizing and head portions of the ultrasound probe configured to keep the power-and-data cable away from the head portion of the ultrasound probe.
19. The method of clause 18, wherein moving the ultrasound probe by the finger-grip portion of the ultrasound probe does not overbend the power-and-data cable due to attenuation of a bending radius of the power-and-data cable by a cable boot having a distal-end portion disposed in an opening of the cable conduit from which the power-and-data cable extends.
20. The method of any clause of clauses 13-19, further comprising enabling reflected ultrasound signals to be reflected from the patient into the array of piezoelectric transducers within the ultrasound probe for conversion into electrical signals for corresponding ultrasound images.

## Claims

1. An ultrasound probe (40), comprising:
a body (102);
a lens (108) disposed in a cutout of the body designating a head portion of the ultrasound probe, the lens configured to pass ultrasound signals therethrough;
a stabilizing portion (146) composed of a longitudinal extension of the body extending away from the head portion of the ultrasound probe, the stabilizing portion of the ultrasound probe configured to stabilize the ultrasound probe on a skin surface of a patient without assistance from a user of the ultrasound probe; and
a finger-grip portion (110) composed of a generally transverse extension of the body extending away from the head portion of the ultrasound probe, the finger-grip portion of the ultrasound probe configured to enable the user to grasp and maneuver the ultrasound probe during use thereof with no more than two or three fingers of a single hand.

2. The ultrasound probe of claim 1, further comprising a cable conduit (194) composed of a curved extension of the body extending away from both the stabilizing and head portions of the ultrasound probe, the cable conduit configured to house a distal-end portion of a power-and-data cable for connecting the ultrasound probe to a console and keep the power-and-data cable away from the head portion of the ultrasound probe.

3. The ultrasound probe of claim 2, further comprising a cable boot (195) having a distal-end portion disposed in an opening of the cable conduit, the cable boot configured to house the distal-end portion of the power-and-data cable proximal of the cable conduit and keep the power-and-data cable farther away from the head portion of the ultrasound probe.

4. The ultrasound probe of claim 3, wherein the cable boot (195) is configured to attenuate a bending radius of the power-and-data cable about the opening of the cable conduit, thereby reducing a risk of damaging the power-and-data cable at the opening of the cable conduit where the power-and-data cable is most likely to overbend.

5. The ultrasound probe of any claim of claims 1-4, wherein the stabilizing portion of the ultrasound probe includes a latitudinal channel (147) extending from side to side of two longitudinal sides of the ultrasound probe, thereby reducing a surface area of a generally flat surface of the stabilizing portion intended to face the skin surface of the patient.

6. The ultrasound probe of claim 5, wherein the surface area of the stabilizing portion of the ultrasound probe reduced by the latitudinal channel facilitates movement of the ultrasound probe across the skin surface of the patient by reducing contact area between the surface of the stabilizing portion and the skin surface of the patient; and/or wherein the surface area of the stabilizing portion of the ultrasound probe reduced by the latitudinal channel reduces suction between the surface of the stabilizing portion and the skin surface of the patient when gel is therebetween and the ultrasound probe is removed from the patient.

7. The ultrasound probe of any claim of claims 1-6, wherein the finger-grip portion of the ultrasound probe includes a concave surface opposite a generally flat surface; and optionally wherein the concave surface of the finger-grip portion is an extension of a concave surface of the stabilizing portion of the ultrasound probe intended to face away from the skin surface of the patient.

8. The ultrasound probe of any claim of claims 1-7, wherein the ultrasound probe is bilaterally symmetrical about a plane of symmetry of the ultrasound probe; and optionally wherein at least a portion of the body is molded thermoplastic coupled together along the plane of symmetry of the ultrasound probe.

9. An ultrasound system, comprising:
a console (20) configured for processing and depicting ultrasound images on a display (30) of the console; and
the ultrasound probe according to claim 1, the ultrasound probe further including:
a cable conduit (194) composed of a curved extension of the body extending away from both the stabilizing and head portions of the ultrasound probe, the cable conduit configured to house a distal-end portion of a power-and-data cable (104) for connecting the ultrasound probe to the console; and

10. A method of using an ultrasound probe (10), comprising:
obtaining the ultrasound probe (10), the ultrasound probe including:
a body (102) having a lens (108) disposed in a cutout of the body designating a head portion of the ultrasound probe,
a stabilizing portion (146) composed of a longitudinal extension of the body extending away from the head portion of the ultrasound probe, and
a finger-grip portion (110) composed of a generally transverse extension of the body extending away from the head portion of the ultrasound probe;
placing the head and stabilizing portions of the ultrasound probe on a skin surface of a patient;
enabling probing ultrasound signals to be emitted into the patient from an array of piezoelectric transducers within the ultrasound probe for ultrasound imaging; and
moving the ultrasound probe for the ultrasound imaging by the finger-grip portion of the ultrasound probe using no more than two or three fingers of a single hand.

11. The method of claim 10, further comprising grasping the ultrasound probe before moving the ultrasound probe, the grasping including placing at least one of an index finger or a middle finger on a concave surface of the finger-grip portion (110) of the ultrasound probe and a thumb on an opposing, generally flat surface of the finger-grip portion of the ultrasound probe; and optionally wherein grasping the ultrasound probe orients a palm of the single hand substantially parallel to the skin surface of the patient.

12. The method of any claim of claims 10 or 11, further comprising applying an ultrasound gel to the skin surface of the patient before placing the head and stabilizing portions of the ultrasound probe on a skin surface of a patient; and optionally further comprising removing the ultrasound probe from the skin surface of a patient, the stabilizing portion of the ultrasound probe including a latitudinal channel extending from side to side of two longitudinal sides of the ultrasound probe that reduces a surface area of a generally flat surface of the stabilizing portion, thereby reducing gel-induced suction between the surface of the stabilizing portion and the skin surface of the patient when removing the ultrasound probe from the skin surface of the patient.

13. The method of any claim of claims 10-12, further comprising connecting a power-and-data cable (104) of the ultrasound probe to a console (20) of an ultrasound imaging system, the cable extending from a cable conduit of the ultrasound probe composed of a curved extension of the body extending away from both the stabilizing and head portions of the ultrasound probe configured to keep the power-and-data cable away from the head portion of the ultrasound probe.

14. The method of claim 13, wherein moving the ultrasound probe by the finger-grip portion of the ultrasound probe does not overbend the power-and-data cable due to attenuation of a bending radius of the power-and-data cable by a cable boot having a distal-end portion disposed in an opening of the cable conduit from which the power-and-data cable extends.

15. The method of any claim of claims 10-14, further comprising enabling reflected ultrasound signals to be reflected from the patient into the array of piezoelectric transducers within the ultrasound probe for conversion into electrical signals for corresponding ultrasound images.
